# EUROPEAN PATENT SPECIFICATION

(11) **EP 3 215 132 B1**
(45) Date of publication and mention of the grant of the patent: **27.01.2021**
(21) Application number: 15857591.0
(22) Date of filing: 03.11.2015
(51) Int. Cl.: A61K 31/13, A61K 9/48, A61K 9/50, A61P 21/00

(54) **AMANTADINE FOR IMPROVING GAIT IN MULTIPLE SCLEROSIS**
AMANTADIN ZUR VERBESSERUNG DES GANGS BEI MULTIPLER SKLEROSE
AMANTADINE POUR L'AMÉLIORATION DE LA MARCHE DANS LA SCLÉROSE EN PLAQUE

(30) Priority: 04.11.2014 US 201462075137 P
(43) Date of publication of application: 13.09.2017
(73) Proprietor: Adamas Pharmaceuticals, Inc., Emeryville, CA 94608 (US)
(72) Inventor: WENT, Gregory T., Mill Valley, California 94941 (US); FULTZ, Timothy J., Jasper, Georgia 30143 (US); NGUYEN, Jack, Berkeley, California 94705 (US); CHERNOFF, David, Sausalito, California 94965 (US)
(74) Representative: Carpmaels & Ransford LLP
(86) International application number: PCT/US2015/058872
(87) International publication number: WO 2016/073510

(56) References cited:
- WO-A1-2014/093475
- WO-A1-2014/204933
- WO-A2-2006/053067
- US-A1- 2008 279 819
- US-A1- 2011 189 273
- US-A1- 2014 179 797
- CHAN HIU-FAI ET AL: "Amantadine improves gait in PD patients with STN stimulation", PARKINSONISM AND RELATED DISORDERS, ELSEVIER SCIENCE, OXFORD, GB, vol. 19, no. 3, 6 December 2012 (2012-12-06), pages 316-319, XP028984445, ISSN: 1353-8020, DOI: 10.1016/J.PARKRELDIS.2012.11.005
- CORNELIU C. LUCA ET AL: "4-Aminopyridine improves freezing of gait in Parkinson's disease", JOURNAL OF NEUROLOGY - ZEITSCHRIFT FUER NEUROLOGIE, vol. 260, no. 10, 30 August 2013 (2013-08-30), pages 2662-2664, XP055472353, DE ISSN: 0340-5354, DOI: 10.1007/s00415-013-7090-0
- RAJESH PAHWA ET AL.: "Amantadine Extended Release for Levodopa-induced Dyskinesia in Parkinson's Disease (EASED Study)", MOVEMENT DISORDERS, vol. 30, no. 6, 4 February 2015 (2015-02-04), pages 788-795, XP009505171,
- Adamas Pharmaceuticals, Inc: "Safety and Efficacy of ADS-5102 in Patients With Multiple Sclerosis and Walking Impairment", , 15 June 2015 (2015-06-15), XP002780811, Retrieved from the Internet: URL:https://clinicaltrials.gov/ct2/show/NC T02471222 [retrieved on 2018-05-04]
- Adamas Pharmaceuticals, Inc.: "Extended Release Amantadine Safety and Efficacy Study in Levodopa-Induced Dyskinesia (EASED Study) (EASED)", , 19 July 2011 (2011-07-19), XP002780812, Retrieved from the Internet: URL:https://clinicaltrials.gov/ct2/show/NC T01397422 [retrieved on 2015-05-04]
- NICHOLAS RICHARD ET AL: "Multiple sclerosis", CLINICAL EVIDENCE, BMJ, UK, vol. 2012, 10 February 2012 (2012-02-10), XP009519419, ISSN: 1752-8526

## Description

### CROSS-REFERENCE

This application claims benefit of priority under 35 U.S.C. § 119(e) from United States Provisional Application No. 62/075,137, filed November 4, 2014.

### BACKGROUND OF THE INVENTION

Amantadine is indicated for various conditions that can be treated by NMDA receptor antagonists including the treatment of idiopathic Parkinson's disease (Paralysis Agitans), post-encephalitic Parkinsonism, and symptomatic Parkinsonism which may follow injury to the nervous system by carbon monoxide intoxication. Amantadine also has activity as a viral M2 channel inhibitor and is used for the prophylaxis and treatment of infection of viral diseases, especially influenza A virus.

Currently marketed forms of amantadine are immediate release formulations that are typically administered two or more times a day. Amantadine's use is limited by dose related CNS side effects including dizziness, confusion, hallucinations, insomnia and nightmares. Gracies JM, Olanow CW; Current and Experimental Therapeutics of Parkinson's Disease; Neuropsycho-pharmacology: the Fifth Generation of Progress, pp. 1802; American College of Neuropsycho-pharmacology 2002), which can be particularly exacerbated when amantadine is administered late in the day (Jackson et al., Bull. Pan Am. Health Org., 147, 595-603 (1967); Jackson, JAMA, 235(25), (1976), pp. 2739-2742; and Hayden, AAC, 23(3) 1983, pp. 458-464.

Immediate release amantadine has demonstrated an improvement in walking speed in Parkinson's patients. Parkes et al., Lancet, 1, pp. 259-62 (1970). Advanced Parkinson's patients with STN stimulation were evaluated in an open label study with immediate release amantadine which showed improvements in gait. Chan et al., Parkinsonism and Related Disorders, 19, pp. 316-9 (2013). Methylphenidate also showed an improvement in gait in Parkinson's patients with severe gait disorders who were receiving STN stimulation. Moreau et al., Lancet Neurology 11, pp. 589-96 (2012). Another study of gait impairment in Parkinson's disease concluded that methylphenidate was not effective. Espay et al., Neurology 76, pp. 1256-62 (2011). In multiple sclerosis, fampridine (4-aminopyridine; dalfampridine) demonstrated improvement in walking ability in some patients. Goodman et al., Lancet, 373, pp. 732-8 (2009). In contrast, fampridine did not show an improvement in Parkinson's patients walking ability (i.e., velocity and stride length) at four weeks (Luca et al., Neurology 84(14) Supplement P.6.049 (2015)). Chan et al. discloses use of amantadine in patients with advanced Parkinson's disease and who have been administered with subthalamic nucleus deep brain stimulation (STN-DBS). US 2011/189273 discloses the use of amantadine in i.a. Parkinson's disease and also multiple sclerosis.

It is known that immediate release amantadine can act as a stimulant, causing insomnia and sleep disturbance. Therefore, the last dose of immediate release amantadine is typically administered no later than 4 pm in order to minimize these side effects. Such dosing of amantadine results in peak plasma amantadine concentrations occurring in the evening or night, and very low plasma concentrations in the morning.

Extended release forms of amantadine have been described in the art. U.S. Patent No. 5,358,721, to Guittard et al., U.S. Patent No. 6,217,905, to Edgren et al., and U.S. Patent No. 8,574,626, to Vergez et al, each disclose an oral osmotic dosage form comprising an antiviral or anti-Parkinson's drug, respectively, where in each case amantadine is listed as a possible drug to be utilized in the dosage form. U.S. Patent No. 6,194,000, to Smith et al., discloses analgesic immediate and controlled release pharmaceutical compositions utilizing NMDA receptor antagonists, such as amantadine, as the active agent. Each of U.S. Patent Appl. Publication Nos. US 2006/0252788, US 2006/0189694, US 2006/0142398, US 2008/0227743, and US 2011/0189273 (each to Went et al.) discloses the administration of an NMDA receptor antagonist, such as amantadine, optionally in controlled release form.

There are eight basic pathological gaits that can be attributed to neurological conditions: hemiplegic, spastic diplegic, neuropathic, myopathic, Parkinsonian, choreiform, ataxic (cerebellar) and sensory. Hemiplegic gait is most commonly seen in stroke. Diplegic gait is seen in bilateral periventricular lesions, such as those seen in cerebral palsy. Neuropathic gait, if bilateral, is seen in amyotrophic lateral sclerosis, Charcot-Marie-Tooth disease and other peripheral neuropathies including those associated with uncontrolled diabetes. Myopathic gait is seen in patient with myopathies, such as muscular dystrophy. Parkinsonian gait is seen in Parkinson's disease or any other condition causing Parkinsonism, such as side effects from drugs. Choreiform gait is seen with certain basal ganglia disorders including Sydenham's chorea, Huntington's disease and other forms of chorea, athetosis or dystonia. Ataxic gait is most commonly seen in cerebellar disease. Patients with multiple sclerosis frequently express ataxic symptoms, including uncoordinated walking, reduced control of range of movement, inability to maintain a steady posture, inability to maintain a steady rhythm, or loss of balance. Sensory gait can be seen in disorders of the dorsal columns (e.g., B 12 deficiency or tabes dorsalis) or in diseases affecting the peripheral nerves such as uncontrolled diabetes.

### Summary of the Invention

The present invention is a dose comprising (i) 220 mg to 600 mg of amantadine or pharmaceutically acceptable salts thereof and (ii) at least one excipient, for use in a method of improving gait in a human subject with multiple sclerosis, the method comprising orally administering the dose to said subject once daily. The inventors have found surprisingly that amantadine is useful in the treatment of certain hypokinetic disorders, including walking impairment or gait impairment in patients with multiple sclerosis or patients who have experienced a stroke. In some embodiments immediate release forms of amantadine or a pharmaceutically acceptable salt thereof or controlled release forms of amantadine or a pharmaceutically acceptable salt thereof are useful in said methods. In some embodiments, controlled release forms are preferred. In one embodiment, the methods include administration of immediate release forms of amantadine or pharmaceutically acceptable salts (including, for example, amantadine hydrochloride or amantadine sulfate) one, two, or three times per day. In one embodiment, the methods include administration of extended release compositions of amantadine or pharmaceutically acceptable salts thereof once daily in the morning (e.g., after the patient awakens and not later than 12 pm) wherein the extended release composition provides a single dose Tmax of 5 to 11 hours. In some embodiments, the methods include administration of extended release compositions of amantadine or pharmaceutically acceptable salts thereof once daily in the evening (e.g., 0 to 4 hours before bedtime) wherein the extended release composition provides a single dose Tmax of 9 to 19 hours. In some embodiments the invention has been found to facilitate once daily administration of a relatively high dose of amantadine (or a pharmaceutically acceptable salt thereof), while surprisingly reducing side effects such as sleep disturbance.

Some embodiments described herein provide a dose of the invention for use in a method of improving walking (e.g., increasing walking speed), or treating impairment of walking in a patient (e.g., an MS patient or a patient who has experienced a stroke). In some embodiments of the disclosure, the method includes administering to the patient about 50 mg to about 600 mg per day of a drug selected from the group consisting of amantadine or a pharmaceutically acceptable salt thereof. In some embodiments of the disclosure, the drug is administered twice daily or three times daily. In the invention the drug is administered once daily. In some embodiments the drug is in a pharmaceutical composition comprising the drug and at least one excipient, such as a sustained release excipient. In some embodiments, the drug is administered in one, two, three or four dosage forms consisting of the drug and at least one excipient. In some embodiments the one, two, three or four dosage forms are oral dosage forms. In some embodiments, the oral dosage forms are tablets, caplets, gel capsules or other suitable oral unit dosage forms. In some embodiments, the patient has normal renal function and the drug is administered at an initial dosage of about 100 to about 220 mg per day, about 120 to about 200 mg per day, about 140 to about 180 mg per day, or about 100, 110, 120, 130, 140, 150, 160, 170, 180, 190, 200, 210 or 220 mg per day. In some embodiments, the patient has normal renal function and the drug is administered, after an initial period of amantadine administration, at a dosage of about 200 to about 440 mg per day, about 240 to about 400 mg per day, about 280 to about 360 mg per day, or about 160, 170, 180, 200, 220, 240, 260, 280, 300, 320, 340, 360, 380, 400, 420 or 440 mg per day. In some embodiments, the patient has renal impairment and the drug is administered at an initial dosage of about 50 to about 110 mg per day, about 60 to about 100 mg per day, about 70 to about 90 mg per day, or about 50, 55, 60, 65, 70, 75, 80, 85, 90, 95, 100, 105 or 110 mg per day. In some such embodiments, the MS patient has renal impairment and, after an initial period of amantadine administration, the dosage is increased to about 100 to about 220 mg per day, about 120 to about 200 mg per day, about 140 to about 180 mg per day, or about 100, 110, 120, 130, 140, 150, 160, 170, 180, 190, 200, 210 or 220 mg per day. In some embodiments the drug is administered in an extended release form comprising the drug and at least one extended release excipient. In some embodiments the drug is administered in the morning, e.g., after waking and not later than 12 pm or between the hours of 5 am and 12 pm, 6 am and 12 pm, 7 am and 12 pm, 8 am and 12 pm, and has a median Tmax of 5 to 8 hours, 5 to 9 hours, 5 to 10 hours, 5 to 11 hours, 6 to 9 hours, 6 to 10 hours, or 6 to 11 hours. In some embodiments the drug is administered before bedtime (e.g., 0, 1, 2, 3 or 4 hours before bedtime) and has a Tmax of 8 to 18 hours, 8 to 20 hours, 10 to 18 hours, 10 to 20 hours, 12 to 18 hours, 12 to 20 hours. The aforementioned references to Tmax are median Tmax values determined from a single dose, fasted, human pharmacokinetic study. In some embodiments, the drug is provided in an immediate release form and administered two, three, or four times daily.

In some embodiments of the disclosure, a method of improving walking in a human subject is provided, said method comprising orally administering to said patient once daily or nightly, a dose comprising (i) 50 mg to 600 mg of a drug selected from the group consisting of amantadine and pharmaceutically acceptable salts thereof and (ii) at least one excipient, (e.g., wherein the dose includes a composition comprising components (i) and (ii)). In the invention the human subject is a patient with MS. In some embodiments, the dose includes a composition comprising (i) 100 mg to 450 mg of amantadine, or a pharmaceutically acceptable salt thereof and (ii) at least one excipient. In some embodiments, the composition may comprise about 130-210 mg of amantadine, or a pharmaceutically acceptable salt thereof. In various specific embodiments, a dosage form containing the composition comprises (i) 50 to 75 mg, 70 to 95 mg, 90 to 115 mg, 110 to 135 mg, 130 to 155 mg, 150 to 175 mg, 170 to 195 mg, 190 to 215 mg, 210 to 235 mg, 230 to 255 mg, 250 to 275 mg, 270 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, 410 to 425 mg, 420 to 435 mg, 430 to 445 mg or 440 to 455 mg of amantadine, or a pharmaceutically acceptable salt thereof, and (ii) at least one excipient. In some embodiments, the composition comprises about 110, 120, 130, 140, 150, 160 170, 180, 190, 210, 220 or 340 mg amantadine, or a pharmaceutically acceptable salt thereof. In some embodiments, the composition comprises 110 mg amantadine hydrochloride. In some embodiments, the composition comprises 130 mg amantadine hydrochloride. In some embodiments, the composition comprises 170 mg amantadine hydrochloride. In some embodiments, the composition comprises 210 mg amantadine hydrochloride. In the invention, the human subject has multiple sclerosis. In some embodiments, the dose additionally comprises one or more drugs selected from the group consisting of 4-aminopyridine, baclofen, dextromethorphan, dimethyl fumarate, fingolimod, methylphenidate, and teriflunomide, tetrabenizine, and tizanidine. In some embodiments, the at least one excipient is a release modifying excipient. In some embodiments, at least one of said excipients modifies the release of the amantadine or pharmaceutically acceptable salt thereof to provide an extended release form, and wherein administration of the dose provides a) a Tmax for amantadine of 5 to 18 hours, and/or b) a Cmax of 1.0 to 2.8 ng/ml per mg amantadine, and/or c) an AUC_{0-inf} of 40 to 75 ng^{∗}hr/ml per mg amantadine as determined from a single dose human pharmacokinetic study. In some embodiments, at least one of said excipients modifies the release of the amantadine or pharmaceutically acceptable salt thereof to provide an extended release form, and wherein administration of the dose provides a) a Tmax for amantadine of 5 to 18 hours, and/or b) a Cmax of 1.0 to 2.8 ng/ml per mg amantadine, and/or c) an AUC_{0-inf} of 40 to 75 ng^{∗}hr/ml per mg amantadine as determined from a single dose human pharmacokinetic study. In some embodiments, such dosing of amantadine results in peak plasma amantadine concentrations occurring in the morning or early afternoon, and very low plasma concentrations in the night. In some embodiments, the drug is provided in an extended release form. In additional specific embodiments, peak amantadine plasma concentration is achieved between 5, 6, 7, 8, 9, 10, 11 or 12 hours to about 9, 10, 11, 12, 13, 14, 15, 16, 17, 18, 19, 20, 21, 22, 23 or 24 hours after administration of a single dose of the composition (e.g., 8 hours to about 18 hours after administration). In some embodiments, the amantadine composition is administered to a patient from 0 to 4 hours prior to bedtime. In some embodiments, the amantadine composition is administered to a patient from 0 to 3, 0 to 2, or 0 to 1 hours prior to bedtime. In some embodiments for once daily, oral administration 0 to 4 hours prior to bedtime, the median Tmax for said composition is 9 to 18 hours. In some embodiments for once daily, oral administration after waking and before 12 pm or between the hours of 6 am and 12 pm, the median Tmax for said composition is 5 to 9 hours. In some embodiments, walking speed or ability is evaluated via at least one of the following or a combination thereof: Timed 25-Foot Walking test (T25FW), Timed Up and Go (TUG), 2 minute walk test, six minute timed walk test (6MTW), and/or, Twelve Item Multiple Sclerosis Walking Scale (MSWS-12) (references provided in Example 10). In some embodiments, walking in the subject is significantly improved. In some embodiments, MS symptoms are improved. In some embodiments T25FW, TUG, 2 minute walk, 6MTW and/or MSWS-12 is significantly improved (relative to placebo). In some embodiments, administration of the composition to a MS disease subject results in a significant reduction in MS disease walking impairment. In a specific embodiment, administration of the composition results in about 5%, 10%, 15%, 20%, 25%, 30%, 35%, or 40%, 45%, 50%, 55%, or 60% reduction in MS disease walking impairment. In a specific embodiment, administration of the composition results in at least 5%, 10%, 15%, 20%, 25%, 30%, 35%, or 40%, 45%, 50%, 55%, or 60% reduction in MS disease walking impairment. In a specific embodiment, administration of the composition results in about 5%, 10%, 15%, 20%, 25%, 30%, 35%, or 40%, 45%, 50%, 55%, or 60% improvement in one or more of the assessment scores mentioned above. In some embodiments, the reduction walking impairment is measured on a numeric scale that is used by or accepted by the FDA or other regulatory agencies to evaluate the effectiveness of and to approve for licensure drugs for the treatment of walking impairment. In some embodiments, the once daily or once nightly composition is administered as one, two, three or four unit dosage forms in unequally or, preferably, equally divided units. In some more specific embodiments, the composition is administered as one, two or three unit dosage forms each comprising 75 to 200 mg amantadine, or a pharmaceutically acceptable salt thereof. In some embodiments, the composition is administered as two unit dosage forms each comprising 100 to 175 mg amantadine, or a pharmaceutically acceptable salt thereof. In some embodiments, the composition is administered as two or three unit dosage forms of unequal, or preferably equal, dosage, each comprising 85 to 250 mg amantadine, or a pharmaceutically acceptable salt thereof. In some embodiments, the composition is administered as two unit dosage forms with equal dosage (e.g., each comprising 130 - 210 mg of amantadine or a pharmaceutically acceptable salt thereof). In some more specific embodiments, the composition is administered as two unit dosage forms each comprising 150 to 180 mg amantadine, or a pharmaceutically acceptable salt thereof.

Some embodiments of the disclosure provide a method of improving gait in a human subject, said method comprising orally administering to said patient once daily or once nightly, a dose comprising (i) 50 mg to 600 mg of a drug selected from the group consisting of amantadine and pharmaceutically acceptable salts thereof and (ii) at least one excipient (e.g., wherein the dose includes a composition comprising components (i) and (ii)). In the invention the human subject has MS. In some embodiments, the composition may comprise 100 mg to 450 mg of amantadine, or a pharmaceutically acceptable salt thereof. In some embodiments, the composition may comprise 130 - 210 mg of amantadine, or a pharmaceutically acceptable salt thereof. In some embodiments, a dosage form containing the composition comprises 50 to 75 mg, 70 to 95 mg, 90 to 115 mg, 110 to 135 mg, 130 to 155 mg, 150 to 175 mg, 170 to 195 mg, 190 to 215 mg, 210 to 235 mg, 230 to 255 mg, 250 to 275 mg, 270 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, 410 to 425 mg, 420 to 435 mg, 430 to 445 mg or 440 to 455 mg of amantadine, or a pharmaceutically acceptable salt thereof. In some embodiments, the composition comprises about 110, 120, 130, 140, 150, 160 170, 180, 190, 210, 220 or 340 mg amantadine, or a pharmaceutically acceptable salt thereof. In some embodiments, the composition comprises 110 mg amantadine hydrochloride. In some embodiments, the composition comprises 130 mg amantadine hydrochloride. In some embodiments, the composition comprises 170 mg amantadine hydrochloride. In some embodiments, the composition comprises 210 mg amantadine hydrochloride. In some embodiments, the human subject has multiple sclerosis. In some embodiments, the dose further comprises one or more drugs selected from the group consisting of 4-aminopyridine, baclofen, dextromethorphan, dimethyl fumarate, fingolimod, methylphenidate, and teriflunomide, tetrabenizine, and tizanidine. In some embodiments, the at least one excipient is a release modifying excipient. In some embodiments, at least one of said excipients modifies the release of the amantadine or pharmaceutically acceptable salt thereof to provide an extended release form, and wherein administration of the dose provides a) a Tmax for amantadine of 5 to 18 hours, b) a Cmax of 1.0 to 2.8 ng/ml per mg amantadine, and c) an AUC_{0-inf} of 40 to 75 ng^{∗}hr/ml per mg amantadine as determined from a single dose human pharmacokinetic study. In some embodiments, such dosing of amantadine results in peak plasma amantadine concentrations occurring in the morning or afternoon, and very low plasma concentrations during the night, i.e., while the patient sleeps. In some embodiments, the drug is provided in an extended release form. In additional specific embodiments, peak amantadine plasma concentration is achieved between 5, 6, 7, 8, 9, 10, 11 or 12 hours to about 9, 10, 11, 12, 13, 14, 15, 16, 17, 18, 19, 20, 21, 22, 23 or 24 hours after administration of a single dose of the composition (e.g., 8 hours to about 18 hours after administration). In some embodiments described herein the amantadine composition is administered to a patient from 0 to 4 hours prior to bedtime. In some embodiments, the amantadine composition is administered to a patient from 0 to 3, 0 to 2, or 0 to 1 hours prior to bedtime. In some embodiments of any of the above aspects, administration of the composition to a MS disease subject results in a significant reduction in MS disease gait impairment. In a specific embodiment, administration of the composition results in at least about 5%, 10%, 15%, 20%, 25%, 30%, 35%, or 40%, 45%, 50%, 55%, or 60% reduction in MS disease gait impairment. In some embodiments, gait (or gait impairment) is measured on a numeric scale that is used by or accepted by the FDA or other regulatory agencies to evaluate the effectiveness of and to approve for licensure drugs for the treatment of gait. In some embodiments, the once daily administration of the amantadine composition (or combination comprising amantadine) provides an improvement in a hypokinetic movement disorder affecting gait. The improvement may be determined by methods known in the art such as timed up and go (TUG), timed 25 foot walk test (T25FW), or six minute timed walk test (6MTW). In some embodiments, the once daily or once nightly composition is administered as one, two, three or four unit dosage forms in unequally or, preferably, equally divided units. In some more specific embodiments, the composition is administered as one unit dosage form comprising 75 to 350 mg amantadine or a pharmaceutically acceptable salt thereof, two or three unit dosage forms each comprising 85 to 175 mg amantadine, or a pharmaceutically acceptable salt thereof. In some embodiments, the composition is administered as two unit dosage forms each comprising 100 to 175 mg amantadine, or a pharmaceutically acceptable salt thereof. In some embodiments, the composition is administered as two or three unit dosage forms of unequal, or preferably equal, dosage, each comprising 85 to 250 mg amantadine, or a pharmaceutically acceptable salt thereof. In some more specific embodiments, the composition is administered as two unit dosage forms each comprising 150 to 180 mg amantadine, or a pharmaceutically acceptable salt thereof. In some embodiments the composition is administered orally, once daily.

The disclosure provides methods of improving hypokinetic movement disorders, generally characterized by decreased bodily movement or muscle rigidity, such as walking, and issues associated with multiple sclerosis and walking deficits following stroke. The disclosure provides methods of improving gait in human subjects with hypokinetic movement disorders associated with conditions such as stroke. These hypokinetic conditions may substantially adversely affect the subjects' ambulation, increasing the disability of the underlying condition.

The disclosure provides methods of improving hyperkinetic movement disorders generally characterized by abnormally heightened, sometimes uncontrollable, movements, such as Huntington's chorea, tardive dyskinesia, and Tourettes' syndrome.

Therefore, there exists a need in the art for improved methods of amantadine therapy for the treatment of motor fluctuations or motor impairments in MS and stroke patients, which can be administered to a patient shortly before they wish to sleep (e.g., at bedtime) without causing insomnia or sleep disturbance. In addition, there is a need for an amantadine therapy which can be taken by the patient before they go to sleep and then provides a suitable plasma concentration of amantadine when they wake up, e.g., in the morning, after a full night's sleep. There exists a need in the art for improved methods of amantadine therapy for the treatment of motor fluctuations or motor impairments in MS and strokes, which can be administered to a patient once daily, upon waking in the morning (e.g., before noon or between the hours of 6 am and 12 pm) to provide said treatment without causing insomnia or sleep disturbance.

Each of the aforementioned disorders, conditions, and diseases result in debilitation of the patient. Thus, there exists a need in the art for methods and compositions for treatment of gait disorders and/or hypokinetic movement disorders, and hyperkinetic disorders.

In some aspects of the invention, the dose of the invention is for use in a method of administering amantadine to a patient in need thereof is provided, said method comprising orally administering certain extended release (ER) compositions comprising amantadine, or a pharmaceutically acceptable salt thereof, less than three hours before bedtime (i.e., the time at which the subject wishes to go to sleep for the night). This aspect also includes the use of such compositions and the use of amantadine, or a pharmaceutically acceptable salt thereof, for the manufacture of a medicament as described below. Alternatively, the composition is administered less than about 4 hours before bedtime. In some aspects, administration occurs less than four, less than three and a half, less than three, less than two and a half, less than two, less than one and a half, less than one or less than half hour before bedtime.

In some aspects of the invention the dose of the invention is for use in a method of administering amantadine to a patient in need thereof, said method comprising orally administering certain ER compositions comprising amantadine, or a pharmaceutically acceptable salt thereof, once daily in the morning after the patient has awakened, preferably before noon (e.g., between the hours of 6 am and 12 pm, 7 am and 12 pm, 8 am and 12 pm). This aspect also includes the use of such compositions and the use of amantadine, or a pharmaceutically acceptable salt thereof, in the manufacture of a medicament as described below.

In some aspects, the invention provides a dose of the invention for use in a method of reducing sleep disturbance in a human subject undergoing treatment with amantadine, said method comprising administering an extended release (ER) composition comprising amantadine, or a pharmaceutically acceptable salt thereof, less than about three hours before bedtime (i.e., the time at which the subject wishes to go to sleep for the night). This aspect also includes the use of such compositions and the use of amantadine for the manufacture of a medicament as described below. Alternatively, the composition is administered less than about 4 hours before bedtime.

In some aspects, the invention provides a dose of the invention for use in a method of reducing sleep disturbance in a human subject undergoing treatment with amantadine, said method comprising administering an extended release (ER) composition comprising amantadine, or a pharmaceutically acceptable salt thereof, once daily in the morning after the subject has awakened. This aspect also includes the use of such compositions and the use of amantadine for the manufacture of a medicament as described below

The dose of the invention comprises 220 mg to 600 mg of amantadine or a pharmaceutically acceptable salt thereof. In some aspects of the invention, amantadine, or a pharmaceutically acceptable salt thereof (such as the hydrochloride) is administered at a reduced amount, i.e., 85 to 260 mg per day, for at least one week prior to once daily administration of the maintenance dose. This titration period may improve tolerability of the maintenance dose. In one aspect of the invention, patients are administered 85 or 170 mg per day for at least one week prior to increasing the dose to 170 or 340 mg per day.

In addition, many patients in need of amantadine therapy have difficulty swallowing. Hence there is a need for amantadine therapy that delivers a therapeutically effective dose of the drug in an oral dosage form that is small in size and does not unduly increase the pill burden. Preferably, such dosage form is administered once per day. Preferably, such dosage form may be sprinkled on and consumed with food, e.g., applesauce.

The dose of the invention is for use in a method of improving gait in a human subject with multiple sclerosis. In some aspects of the disclosure, a method of treating brain injury, brain trauma, stroke, Huntington's disease, ALS, neurodegenerative diseases, cerebrovascular conditions, movement disorders, cranial nerve disorders is provided, said method comprising administering certain extended release (ER) compositions comprising amantadine, or a pharmaceutically acceptable salt thereof, less than about three hours before bedtime (i.e., the time at which the subject wishes to go to sleep for the night). This aspect also includes the use of such compositions and the use of amantadine for the manufacture of a medicament as described below.

In some embodiments of any of the above aspects the patient has multiple sclerosis (e.g., been diagnosed with multiple sclerosis). In some embodiments of the disclosure the patient has experienced a stroke.

In some embodiments of any of the above aspects, the composition is administered once nightly. In another aspect, the daily dose exceeds 200 mg and, preferably, is from 240 to 420 mg, more preferably from 260 to 340 mg (even more preferably, 340 mg). In some embodiments, the daily dose of 260 to 340 mg is given in 1, 2 or 3 capsules of size 0, 1 or 2, in normal and/or EL formats.

In some embodiments of any of the above aspects, administration of the composition to a patient results in a significant improvement in Clinician Global Impression (CGI) or any other physician measurement of a patient's overall condition. In a specific embodiment, administration of the composition results in about 5%, 10%, 15%, 20%, 25%, 30%, 35%, or 40% improvement in CGI. In further specific embodiments, the improvement in CGI is measured on a numeric scale that is used by the FDA to evaluate effectiveness of drugs indicated to treat CNS disorders.

In some embodiments of any of the above aspects, there is no increase in the steady state plasma concentration of amantadine for at least one hour after administration of the dose.

In some embodiments of any of the above aspects, there is no increase in the steady state plasma concentration of amantadine for at least two hours after the administration of the dose.

In some embodiments of any of the above aspects, the administration of the composition to a human subject at steady state amantadine plasma concentrations increases the amantadine plasma concentration by less than 5%, 10%, 15%, 20% or 25% at 1, 2, 2.5 or 3 hours following such administration. For example, administration of the composition to a human subject at steady state amantadine plasma concentrations increases the amantadine plasma concentration by less than 5% at 1, 2, 2.5 or 3 hours following such administration; or by less than 10% at 1, 2, 2.5 or 3 hours following such administration; or by less than 15% at 1, 2, 2.5 or 3 hours following such administration; or by less than 20% at 1, 2, 2.5 or 3 hours following such administration; or by less than 25% at 1, 2, 2.5 or 3 hours following such administration.

In one embodiment of any of the above aspects peak plasma concentration of amantadine is achieved between 5 and 16 hours after administration of a single dose of the composition. In a more specific embodiment, peak amantadine plasma concentration is achieved 8 to 14 hours after administration of a single dose of the composition. In another more specific embodiment, peak amantadine plasma concentration is achieved 10 to 12 hours after administration of a single dose of the composition. In additional specific embodiments, peak amantadine plasma concentration is achieved between 5, 6, 7, 8, 9, 10, 11 or 12 hours to about 9, 10, 11, 12, 13, 14, 15, 16, 17, 18, 19, 20, 21, 22, 23 or 24 hours after administration of a single dose of the composition (e.g., 8 hours to about 18 hours after administration).

In some embodiments of any of the above aspects the amantadine has a single dose Tmax of 9 to 18 hours. In more specific embodiments, the amantadine has a single dose Tmax of 12 to 18 hours after administration.

In some embodiments of any of the above aspects the amantadine has a steady state Tmax of 7 to 13 hours. In more specific embodiments, the amantadine has a steady state Tmax of 8 to 12 hours after administration.

In some embodiments of any of the above aspects, a once nightly oral administration of the composition to a human subject provides a steady state plasma concentration profile characterized by a concentration increase of amantadine of less than 25% at three hours after the administration. In more specific embodiments, the steady state plasma concentration profile is characterized by a concentration increase of amantadine of less than 25% at four hours after the administration.

In some embodiments of any of the above aspects, the composition is administered once a day and the ratio of Cmax to Cmin at steady state is 1.3 to 3.0, 1.3 to 2.6, 1.3 to 2.4, 1.3 to 2.2, 1.4 to 3.0, 1.4 to 2.6, 1.4 to 2.4, 1.4 to 2.2, 1.5 to 3.0, 1.5 to 2.6, 1.5 to 2.4, or 1.5 to 2.2.

In embodiments of any of the above aspects, the steady state plasma concentration profile following multiple administrations to a human subject of the composition once daily is - characterized by an average plasma concentration during the day ("C-ave-day", defined as the average day time amantadine plasma concentration as measured in a human PK study) that is 1.4 to 1.7 times the average plasma concentration during the night ("C-ave-night", defined as the average night time amantadine plasma concentration as measured in a human PK study). In more specific embodiments the C-ave-day is the average amantadine plasma concentration as measured between the hours of 5 am, 6 am, 7 am, 8 am or 9 am to the hours of 4 pm, 5 pm, 6 pm, 7 pm or 8 pm; for example, between the hours of 6 am and 4 pm, between the hours of 7 am and 6 pm, or between the hours of 7 am and 5 pm. The C-ave-night is the average amantadine plasma concentration as measured between the hours of 4 pm, 5 pm, 6pm, 7 pm, 8 pm, 9 pm, 10 pm or 11 pm to the hours of 5 am, 6 am, 7 am, 8 am or 9 am; for example, between the hours of 10 pm and 6 am, between the hours of 7 pm and 6 am, or between the hours of 8 pm and 6 am.

In some embodiments of any of the above aspects the amantadine is administered as a pharmaceutically acceptable salt. In a more specific embodiment, the amantadine is administered as amantadine hydrochloride or amantadine sulfate. In some embodiments of any of the above aspects, the once daily or once nightly dose of amantadine, or pharmaceutically acceptable salt thereof, may be in the range of 260 to 420 mg. In some embodiments of the disclosure, the once daily or once nightly dose of amantadine, or pharmaceutically acceptable salt thereof, may be in the range of 50 to 600 mg. In other embodiments, the once daily or once nightly dose of amantadine, or pharmaceutically acceptable salt thereof, exceeds 300 mg per day, e.g., is between 320 and 360 mg per day, more specifically is between 330 and 350 mg per day. The dose of the invention comprises 220 mg to 600 mg of amantadine or a pharmaceutically acceptable salt thereof. In various specific embodiments, the daily dose of amantadine or pharmaceutically acceptable salt thereof may be 230 to 255 mg, 250 to 275 mg, 270 to 295 mg, 290 to 305, 300 to 315 mg, 310 to 325 mg, 320 to 335 mg, 330 to 345 mg, 340 to 355 mg, or 350 to 365 mg. Daily doses of 50 to 75 mg, 70 to 95 mg, 90 to 115 mg, 110 to 135 mg, 130 to 155 mg, 150 to 175 mg, 170 to 195 mg, 190 to 215 mg, and 210 to 235 mg are also disclosed. In some particularly preferred embodiments, the once daily or once nightly dose of amantadine, or pharmaceutically acceptable salt thereof, is 340 mg. In some embodiments, the once daily or once nightly dose of amantadine, or pharmaceutically acceptable salt thereof, is 360 to 375 mg, 370 to 385 mg, 380 to 395 mg, 390 to 405 mg, 400 to 415 mg, 410 to 425 mg, 420 to 435 mg, 430 to 445 mg or 440 to 455 mg.

In some embodiments of any of the above aspects, the composition comprises 50 mg to 600 mg of amantadine, or a pharmaceutically acceptable salt thereof. In some embodiments, the composition may comprise 100 mg to 450 mg of amantadine, or a pharmaceutically acceptable salt thereof. In some embodiments, the composition may comprise 130 - 210 mg of amantadine, or a pharmaceutically acceptable salt thereof. In various specific embodiments, a dosage form containing the composition comprises 50 to 75 mg, 70 to 95 mg, 90 to 115 mg, 110 to 135 mg, 130 to 155 mg, 150 to 175 mg, 170 to 195 mg, 190 to 215 mg, 210 to 235 mg, 230 to 255 mg, 250 to 275 mg, 270 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, 410 to 425 mg, 420 to 435 mg, 430 to 445 mg or 440 to 455 mg of amantadine, or a pharmaceutically acceptable salt thereof. In some embodiments, the composition comprises about 110, 120, 130, 140, 150, 160 170, 180, 190, 210, or 220 mg amantadine, or a pharmaceutically acceptable salt thereof. In some embodiments, the composition comprises 110 mg amantadine hydrochloride. In some embodiments, the composition comprises 130 mg amantadine hydrochloride. In some embodiments, the composition comprises 170 mg amantadine hydrochloride. In some embodiments, the composition comprises 210 mg amantadine hydrochloride.

In some embodiments of any of the above aspects, the once daily or once nightly composition is administered as one, two, three or four unit dosage forms in unequally or, preferably, equally divided units. In some more specific embodiments, the composition is administered as two or three unit dosage forms each comprising 85 to 175 mg amantadine, or a pharmaceutically acceptable salt thereof. In some embodiments, the composition is administered as two unit dosage forms each comprising 100 to 175 mg amantadine, or a pharmaceutically acceptable salt thereof.

In some embodiments of any of the above aspects, the composition is administered as two or three unit dosage forms of unequal, or preferably equal, dosage, each comprising 85 to 250 mg amantadine, or a pharmaceutically acceptable salt thereof. In some more specific embodiments, the composition is administered as two unit dosage forms each comprising 150 to 180 mg amantadine, or a pharmaceutically acceptable salt thereof.

In some embodiments of any of the above aspects, oral administration of a single dose of the composition to a cohort of human healthy volunteer subjects in a fasted state provides an average maximum plasma concentration (Cmax) of 1.1 to 2.8 ng/ml per mg of amantadine. In more specific embodiments, oral administration of a single dose of the composition to a cohort of human subjects in a fasted state provides an average maximum plasma concentration (Cmax) of 1.4 to 2.6 ng/ml per mg of amantadine and an AUC_{0-inf} (Area under the concentration-curve curve from t = 0 to t = infinity) of 46 to 60 ng^{∗}h/mL per mg of amantadine.

In some embodiments of any of the above aspects, the daily oral administration of a dose of the composition to a cohort of human subjects provides a steady state plasma concentration profile characterized by at least one of: (i) a mean Cmax of 2.2 to 2.7 ng/ml per mg of amantadine, (ii) a mean Cmin of 1.4 to 1.7 ng/ml per mg of amantadine, and (iii) a mean AUC₀₋₂₄ of 46 to 60 ng^{∗}h/mL per mg of amantadine. In more specific examples, all three criteria of (i), (ii) and (iii) are met.

In more specific embodiments, the steady state plasma concentration profile is further characterized by: (iv) no increase in concentration of amantadine for at least one hour after the administration; and (v) Cmax/Cmin ratio of 1.3 to 3.0. In more specific embodiments, both criteria of (iv) and (v) are met.

In some embodiments, the steady state plasma concentration profile is further characterized by at least one of: (iv) no increase in plasma concentration of amantadine for at least two hours after the administration; and (v) a Cmax/Cmin ratio of 1.3 to 3.0. In some embodiments, both criteria of (iv) and (v) are met.

In some embodiments the composition has an in vitro dissolution profile of amantadine which shows at least one of (i) not more than 25% dissolution at 2 hours, (ii) not more 55-85% dissolution at 6 hours, and (iii) at least 80% dissolution at 12 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

In some embodiments, the composition has an in vitro dissolution profile of amantadine which shows at least one of (i) not more than 25% dissolution at 2 hours, (ii) not more than 25-55% dissolution at 6 hours, and (iii) at least 80% dissolution at 12 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

In some embodiments the composition has an in vitro dissolution profile of amantadine which shows at least one of (i) not more than 20% dissolution at 1 hour, (ii) about 25-45% dissolution at 2 hours, (iii) not more than 50-80% dissolution at 4 hours, and (iv) at least 80% dissolution at 8 hours, using a USP Apparatus II (Paddles) at 50 rpm with 500 ml water at 37° C as the dissolution medium. In some embodiments, two of criteria (i), (ii), (iii) and (iv) are met. In some embodiments, all four of criteria (i), (ii), (iii) and (iv) are met.

In some embodiments the in vitro dissolution profile of amantadine is further characterized by release of amantadine of: (i) not more than 10 % at 1 hour, or (ii) 30-50 % at 4 hours, or (iii) at least 90% at 12 hours using a USP Apparatus II (Paddles) at 50 rpm with 500 ml water at 37° C as the dissolution medium. In some embodiments two of criteria (i), (ii) and (iii) are met. In some embodiments, all three criteria of (i), (ii) and (iii) are met.

In some embodiments, the present invention provides a pharmaceutical composition comprising or consisting of a pellet-in-capsule, wherein a pellet comprises a core that comprises a core seed with a mixture of amantadine and a binder coated onto the core seed, and an extended release coating surrounding the core comprising ethyl cellulose, a pore forming agent such as hydroxypropyl methyl cellulose or povidone, and a plasticizer.

In some embodiments, the present invention provides a pharmaceutical composition for use in the methods of the aspects described above, wherein said composition is for oral administration and comprises a capsule for oral administration, said capsule comprising a plurality of pellets, each pellet comprising: (a) a pellet core comprising amantadine, or a pharmaceutically acceptable salt thereof, and (b) an extended release coating surrounding the pellet core.

In some embodiments, the extended release coating comprises ethyl cellulose and at least one of povidone and hydroxypropyl methyl cellulose, and a plasticizer. In some embodiments, the extended release coating comprises ethyl cellulose, povidone, and a plasticizer.

In some embodiments, the pellet core comprises amantadine and a binder coated onto a core seed. In some embodiments, the core seed is a sugar sphere (nonpareil) or microcrystalline cellulose seed (e.g., Celphere®). In some embodiments, the core seed is a microcrystalline cellulose core. In some embodiments, the core seed has a diameter in the range of 100 microns to 1,000 microns (e.g., at least about 95% have this diameter). In some embodiments, the core seed has a diameter of 100, 200, 300, 400, 500, 600 or 700 microns. In some embodiments, the core seed has a diameter of less than 500 microns (e.g., at least about 95% have this diameter).

In some embodiments, based on the combined weight of the pellet core and extended release coating, the amantadine, or a pharmaceutically acceptable salt thereof, is present in amounts from 20 to 80 wt %, with a bulk density of 0.3 to 1.2 g/cm³.

In some embodiments, based on the combined weight of the pellet core and extended release coating, the amantadine, or a pharmaceutically acceptable salt thereof, is present in amounts from 40 to 60 wt %, with a bulk density of 0.5 to 1.2 g/cm³.

In some embodiments, based on the combined weight of the pellet core and extended release coating, the amantadine, or a pharmaceutically acceptable salt thereof, is present in amounts from 60 to 80 wt %, with a bulk density of 0.5 to 1.2 g/cm³.

In some embodiments, based on the combined weight of the pellet core and extended release coating, the binder is present in amounts from 8 to 25 wt %.

In some embodiments, based on the combined weight of the pellet core and extended release coating, the core seed is present in amounts from 8 to 25 wt %.

In some embodiments, based on the combined weight of the pellet core and extended release coating, the ethyl cellulose is present in amounts from 10 to 20 wt %.

In some embodiments, based on the combined weight of the pellet core and extended release coating, the povidone is present in amounts from 1 to 4 wt %.

In some embodiments, based on the combined weight of the pellet core and extended release coating, and the plasticizer is present in amounts from 1 to 4 wt %.

In some embodiments, the coated pellet has a diameter in the range of 200 microns to 1700 micros. In some embodiments, the coated pellet has a diameter of 200, 300, 400, 500, 600, 700, 800, 900, 1000, 1100, 1200, 1300 or 1500 microns. In some embodiments, the coated pellet has a diameter of less than 1000 microns, e.g., from 500 to 1000 microns.

In some embodiments, based on the combined weight of the pellet core and extended release coating, the binder is present in amounts from 5 to 25 wt %.

In some embodiments, based on the combined weight of the pellet core and extended release coating, the core seed is present in amounts from 1 to 15 wt %.

In some embodiments, based on the combined weight of the pellet core and extended release coating, the ethyl cellulose is present in amounts from 5 to 20 wt %.

In some embodiments, based on the combined weight of the pellet core and extended release coating, the povidone is present in amounts from 0.25 to 4 wt %.

In some embodiments, based on the combined weight of the pellet core and extended release coating, and the plasticizer is present in amounts from 0.25 to 4 wt %.

In some embodiments, the pellet further comprises a seal coating between the pellet core and the extended release coating. In some embodiments, an inert coating can be applied to the inert core prior to drug coating or on drug -coated pellets or on controlled release coated pellets. In another embodiment, an enteric coating can be applied to the drug coated pellets or controlled release pellets.

In some embodiments, the pellet core comprises a binder, selected from the group consisting of hydroxypropyl methyl cellulose, copovidone, and mixtures thereof.

In some embodiments, the above composition is provided in a size 3, size 2, size 1, size 0 or size 00 capsule, including EL forms of these capsule sizes.

In some embodiments, the therapeutically effective daily dose of the above composition is administered in no more than two capsules. In another embodiment, the therapeutically effective daily dose of the composition is administered in no more than three size 1 capsules. In another embodiment, the therapeutically effective daily dose of the composition is administered in no more than two size 0 capsules. In a still more preferred embodiment, the therapeutically effective daily dose of the composition is administered in no more than two size 1 capsules. In another embodiment, the therapeutically effective daily dose of the composition is administered in no more than three size 2 capsules.

In a preferred embodiment, the above composition is provided in an amount of 50 to 110 mg of amantadine or a pharmaceutically acceptable salt thereof in a size 2 capsule, and in the amount of 110 mg to 210 mg of amantadine or a pharmaceutically acceptable salt thereof in a size 1 capsule. In additional embodiments, the above composition comprises coated pellets of diameter 300 to 1000 microns (e.g., at least about 95% have this diameter), with amantadine or pharmaceutically acceptable salt thereof content of 40-80% wt % and at a bulk density of 0.5-1.2 g/cm³. In a further preferred embodiment, the above composition has an in vitro dissolution profile of amantadine which shows at least one of (i) not more than 25% dissolution at 2 hours, (ii) not more than 55-85% dissolution at 6 hours, and (iii) at least 80% dissolution at 12 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

In some embodiments, the plasticizer is selected from the group consisting of medium chain triglycerides, diethyl phthalate, citrate esters, polyethylene glycol, glycerol, acetylated glycerides, and castor oil. In some embodiments, the plasticizer is medium chain triglycerides, e.g., Miglyol 812 N.

In some embodiments, the present invention provides a dose for use in a method of administering amantadine, or a pharmaceutically acceptable salt thereof, to a human subject in need thereof, said method comprising orally administering a composition of any of the above aspects.

In a preferred aspect, the present invention provides a dose for use in a method of treating disease in a human subject in need thereof, said method comprising orally administering a composition of any of the above aspects once daily at nighttime, administering 1, 2 or 3 capsules or dosage forms.

In a preferred aspect, the present invention provides a dose for use in a method of treating disease in a human subject in need thereof, said method comprising orally administering a composition of any of the above aspects once daily in the morning, administering 1, 2 or 3 capsules or dosage forms.

References to administering amantadine to a subject in need thereof include treating a patient with a disease or condition, including an iatrogenic condition, which may be treated, prevented or cured by a NMDA antagonist. More specifically, administering amantadine to a subject in need thereof includes treating a patient with brain injury, brain trauma, stroke (and walking deficits associated therewith), Huntington's disease (and chorea associated therewith), ALS, Multiple Sclerosis (and walking issues associated therewith), neurodegenerative diseases, cerebrovascular conditions, movement disorders, cranial nerve disorders, Tourette's syndrome, tardive dyskinesia, and other CNS disorders.

Some embodiments described herein provide a dose of the invention for use in a method of improving CGI in a patient, comprising administering to said patient once nightly, 0 to 4 hours before bedtime a composition comprising 260 to 420 mg amantadine, or a pharmaceutically acceptable salt thereof, and at least one release modifying excipient. In some such embodiments, the dose of the invention for use in a method can be for treatment of the disorders and conditions described herein. In some embodiments, the composition comprises 260 to 340 mg amantadine, or a pharmaceutically acceptable salt thereof. In some embodiments, the composition comprises 260 mg amantadine, or a pharmaceutically acceptable salt thereof. In some embodiments, the composition comprises 340 mg amantadine, or a pharmaceutically acceptable salt thereof. In some embodiments, the change in CGI is determined in a placebo controlled, double blind clinical study.

### Brief Description of the Drawings

Figure 1 shows a plot of mean (SD) plasma amantadine concentrations versus scheduled time for Formulation 1 in a single dose, fasted, PK study (Example 4).
Figure 2 shows the simulated mean plasma concentration of amantadine versus time curves following multiple dose administration of various strengths of amantadine ER administered once nightly. (Example 5).
Figure 3 shows the dissolution profiles for three amantadine ER formulations, A, B and C, referred to in Example 7.
Figure 4 shows a plot of mean (SD) plasma amantadine concentrations versus scheduled time for four (4) amantadine treatments.
Figure 5 shows a semi-logarithmic mean (SD) plasma amantadine concentrations versus scheduled time for four (4) amantadine treatments.
Figure 6 shows the clinical scores of the 4 test groups used in the MotoRater assay. (Example 9).
Figure 7 shows a scatter plot of the cumulative clinical scores of the 4 test groups used in the MotoRater assay. (Example 9).
Figure 8 shows a scatter plot of the cumulative clinical scores of the Iba-1 expression by each of the 4 test groups used in the MotoRater assay. (Example 9).
Figure 9 shows a line graph depicting the % of animals in each test group that were capable of completing the MotoRater walk test during the 27 day study. (Example 9).
Figure 10A provides a bar graph showing the time to walk 60 cm on Day 14 of the study. Figure 10B shows the mean speed for each group on Day 14.

### Detailed Description of the Invention

The dose of the invention is, in one embodiment, for use in a method which comprises orally administering to the patient an extended release (ER) amantadine composition designed for nighttime administration. The composition is taken less than three hours before bedtime, and preferably less than two and a half, less than two, less than one and a half, or less than one hour before bedtime. Most preferably the ER amantadine composition is taken less than half hour before bedtime (i.e., the time at which the subject wishes to go to sleep for the night). Alternatively, the composition is administered less than about 4 hours before bedtime. The dose of the invention is, in one embodiment, for use in a method which comprises orally administering to the patient an extended release (ER) amantadine composition designed for morning administration. The composition is taken after waking, preferably before noon, more preferably between the hours of 6 am and 12 pm, 7 am and 12 pm, 8am and 12 pm, 6 am and 11 am, 7 am and 11 am.

The dose of the invention is, in one embodiment, for use in a method which comprises orally administering to the patient an immediate release amantadine composition, one times per day.

As used herein, a reference to amantadine is intended to encompass pharmaceutically acceptable salts thereof (e.g., amantadine hydrochloride, amantadine sulfate, etc.).

As used herein, "extended release" includes "controlled release", "modified release", "sustained release", "timed release", "delayed release", and also mixtures of delayed release, immediate release, enteric coated, etc., with each of the above.

The patient may be diagnosed with any disease or disorder for which amantadine is prescribed. In some embodiments the present methods are for preventing the diseases and disorders described herein, e.g., when a patient is at risk of developing the diseases and disorders. In some embodiments of the disclosure, the patient has a brain injury, brain injury, brain trauma, stroke, Huntington's disease, ALS, neurodegenerative diseases, cerebrovascular conditions, movement disorders, tardive dyskinesia, Tourette's syndrome, or cranial nerve disorders.

The invention also provides a dose for use in a method of reducing sleep disturbances in a patient undergoing treatment with amantadine. The method comprises administering amantadine to a patient in need thereof, such that the amantadine does not interfere with sleep, yet provides maximum benefit in morning hours when often needed most by many patients who take amantadine and further, provides nighttime coverage of symptoms of the disease if needed. Nighttime coverage includes providing benefit if the patient wakes up and wishes to return to sleep. In some such methods, the C-ave-day is 1.2 to 1.7 times the C-ave-night; in preferred methods the C-ave-day is measured between the hours of 8 am to 8 pm and the C-ave-night is measured between the hours of 8 pm to 8 am. In some such methods, administration of a single dose of the composition to a cohort of human healthy volunteer subjects in a fasted state provides an average maximum plasma concentration (Cmax) of 1.1 to 2.2 ng/ml per mg of amantadine or an AUC_{0-inf} (Area under the concentration-curve curve from t = 0 to t = infinity) of 46 to 56 ng^{∗}h/mL per mg of amantadine or both. In some such methods, the daily oral administration of a dose of the composition to a cohort of human subjects provides a steady state plasma concentration profile characterized by at least one of: (i) a mean Cmax of 2.2 to 3.0 ng/ml per mg of amantadine, (ii) a mean Cmin of 1.4 to 1.7 ng/ml per mg of amantadine, and (iii) a mean AUC₀₋₂₄ of 46 to 56 ng^{∗}h/mL per mg of amantadine; in more specific methods, all three criteria of (i), (ii) and (iii) are met.

An ER amantadine composition for use in the invention is adapted for nighttime administration by providing a plasma concentration profile that does not interfere with the subject's sleep. The composition of the invention will, upon administration to a human subject, result in a gradual initial increase in plasma concentration of amantadine such that, at steady state conditions, administration of a dose of the composition results in an increase in plasma concentration of amantadine of less than 25% at three hours after the dose is administered. For example, if a subject's steady state plasma concentration of amantadine is 500 ng/ml at the time a dose of the composition is administered, three hours later the subject's plasma concentration of amantadine will be less than 625 ng/ml. Preferably, the increase in plasma concentration of amantadine three hours after administration is less than 15%, and most preferably, less than 10%. Particularly preferred compositions have a plasma concentration profile further characterized by no increase in amantadine plasma concentration, or even a decrease (at steady state conditions), for at least one or, in a preferred embodiment, two hours after the administration. The composition for use in the invention is further adapted for bedtime (i.e., the time at which the subject wishes to go to sleep for the night) administration by providing a maximum concentration of amantadine (Cmax) in the morning hours. The time to reach Cmax (Tmax), as measured after single dose administration in the fasted state, is in some embodiments at least, 9 hours and up to 13, 14, 15, 16, 17, or 18 hours, or at least 10hours and up to 14, 15, 16, 17, or 18 hours, or at least 12 hours, and up to 14, 15, 16, or 17 hours. In specific embodiments, the Tmax is 9 to 18 hours, most preferably 12 to 18 hours. In some embodiments Tmax is 8 to 18 hours. At steady state, with once nightly administration of the composition, the Tmax can be 7 to 13 hours, most preferably 8 to 12 hours.

A suitable ER amantadine composition may be further characterized by having a steady-state Cmax/Cmin ratio of 1.3 to 3.0, and preferably 1.5 to 2.8, resulting in a composition with a daily profile exhibiting a diurnal peak in the daytime or waking hours and a trough in the nighttime or sleeping hours. Preferred ER amantadine compositions provide increased diurnal variation, (i.e., increased Cmax/Cmin ratio) while providing steady state Cmax during the daytime hours (e.g., 9 am to 6 pm, 9 am to 5 pm, 9 am to 4 pm, 10 am to 6 pm, 10 am to 5 pm, 10 am to 4 pm).

In more specific, preferred embodiments, the plasma concentration profile is further characterized by having an AUC profile after administration of a single dose of the composition characterized by: a fractional AUC from 0 to 4 hours that is less than 5%, and preferably less than 3% of AUCo-inf; a fractional AUC from 0 to 8 hours that is about 5 to 15%, and preferably about 8 to 12% of AUCo-inf; a fractional AUC from 0 to 12 hours that is about 10 to 40%, and preferably about 15 to 30% of AUCo-inf; a fractional AUC from 0 to 18 hours that is about 25 to 60%, and preferably about 30 to 50% of AUC_{0-inf}; and a fractional AUC from 0 to 24 hours that is about 40 to 75%, and preferably about 50 to 70% of AUC_{0-inf}.

In a further preferred embodiment, the plasma concentration profile is further characterized by having an AUC profile after once nightly dosing of the composition at steady state conditions characterized by: a fractional AUC from 0 to 4 hours that is about 2 to 25%, and preferably about 5 to 20% of AUC₀₋₂₄; a fractional AUC from 0 to 8 hours that is about 15 to 50%, and preferably about 20 to 40% of AUC₀₋₂₄; a fractional AUC from 0 to 12 hours that is about 30 to 70%, and preferably about 40 to 60% of AUC₀₋₂₄: and a fractional AUC from 0 to 18 hours that is about 60 to 95%, and preferably about 75 to 90% of AUC₀₋₂₄.

In some embodiments of any of the above aspects, the steady state plasma concentration profile following multiple administrations to a human subject of the composition at once daily is characterized by an average plasma concentration during the day ("C-ave-day", defined as the average day time amantadine plasma concentration as measured in a human PK study) that is 1.1 to 2.0 times the average plasma concentration during the night ("C-ave-night", defined as the average night time amantadine plasma concentration as measured in a human PK study). In some embodiments, the ratio of C-ave-day/C-ave-night at steady state is within one of the ranges 1.1 to 1.9, 1.1 to 1.8, 1.1 to 1.7, 1.1 to 1.6, 1.1 to 1.5, 1.1 to 1.4, 1.2 to 1.9, 1.2 to 1.7, 1.2 to 1.6, 1.2 to 1.5,1.3 to 1.9, 1.3 to 1.8, 1.3 to 1.7, 1.3 to 1.6, 1.4 to 1.9, 1.4 to 1.8, 1.4 to 1.7, 1.5 to 1.9, 1.5 to 1.8, 1.5 to 1.7, 1.6 to 1.8, or 1.6 to 1.9. In some embodiments, the ratio of C-ave-day/C-ave-night at steady state is 1.1, 1.15, 1.2, 1.25, 1.3, 1.35, 1.4, 1.45, 1.5, 1.55, 1.6, 1.65, 1.7, 1.75, 1.8, 1.85, or 1.9. In some embodiments, the C-ave-day is the average amantadine plasma concentration as measured between the hours of 5 am, 6 am, 7 am, 8 am or 9 am to the hours of 4 pm, 5 pm, 6 pm, 7 pm or 8 pm and the C-ave-night is the average amantadine plasma concentration as measured between the hours of 4 pm, 5 pm, 6pm, 7 pm, 8 pm, 9 pm, 10 pm or 11 pm to the hours of 5 am, 6 am, 7 am, 8 am or 9 am. In some embodiments, the C-ave-day is the average amantadine plasma concentration as measured within any four to twelve hour period between the hours of 5 am and 8 pm; and the C-ave-night is the average amantadine plasma concentration as measured within any four to twelve hour period between the hours of 8 pm and 5 am. In some embodiments, the C-ave-day is the average amantadine plasma concentration as measured within any four, five, six, seven, eight, nine, ten, eleven or twelve hour period between the hours of 5 am and 8 pm; and the C-ave-night is the average amantadine plasma concentration as measured within any four, five, six, seven, eight, nine, ten, eleven or twelve hour period between the hours of 8 pm and 5 am.

In some embodiments described herein an amantadine composition is administered to a patient from 0 to 4 hours prior to bedtime. In some embodiments, the amantadine composition is administered to a patient from 0 to 3, 0 to 2, or 0 to 1 hours prior to bedtime. In some embodiments, the amantadine composition is administered to a patient from 0 to 240 minutes, from 0 to 180 minutes, e.g., from 0 to 120 minutes, from 0 to 60 minutes, from 0 to 45 minutes, from 0 to 30 minutes, from 0 to 15 minutes or from 0 to 10 minutes prior to bedtime. In some embodiments, the amantadine composition is administered to a patient from 60 to 240 minutes, from 60 to 180 minutes, from 60 to 120 minutes or from 60 to 90 minutes prior to bedtime.

It is to be understood that administration to a patient includes administration by a healthcare professional and self-administration by the patient.

Unless otherwise specified herein, the term "bedtime" has the normal meaning of a time when a person retires for the primary sleep period during a twenty-four hour period of time. While for the general populace, bedtime occurs at night, there are patients, such as those who work nights, for whom bedtime occurs during the day. Thus, in some embodiments, bedtime may be anytime during the day or night.

As used herein, unless otherwise indicated, reference to a plasma concentration profile or a specific pharmacokinetic property (e.g., Cmax, Cmin, AUC, Tmax, etc.) in a human subject refers to a mean value (or in some embodiments a median value for Tmax) obtained from healthy adults determined in a typical phase I clinical trial designed to measure pharmacokinetic properties of a drug (see, e.g., Examples 2 and 3, below). References herein to Tmax and T1/2 refer to values obtained after administration of a single dose at fasted states, unless otherwise indicated.

In some embodiments of the invention, the dose of the amantadine administered in accordance with the present invention is within or above the ranges normally prescribed for immediate release compositions of amantadine. As described herein, the unit doses of the amantadine administered in accordance with the present invention are generally higher than the ranges normally prescribed for immediate release compositions of amantadine. For example, the recommended dose of amantadine for the treatment of Parkinson's disease is 100 mg immediate release amantadine administered twice daily. In limited cases of the patient not deriving sufficient benefit at that dose and subject to the patient being able to tolerate such higher dose, the daily dose may be increased to 300 mg or 400 mg, which is always administered in divided doses. Prior to the current invention, the most commonly prescribed dose of amantadine is 200 mg per day, always administered in divided doses. Prior to the current invention, more than 200 mg (for example 300 mg) was always given in divided doses. For the present invention, doses of 260 to 420 mg are administered for treatment of MS and stroke patients, and the doses, uses and compositions of the invention may comprise once-nightly administration of a dose as defined by any of these ranges, particularly at doses from 260 mg to 420 mg, and most preferably 340 mg, once nightly. In some such embodiments the administration of such higher doses is at night, i.e., after 4 pm and/or within 4 hours of bedtime. In additional embodiments the administration of such higher doses may be in the form of 1, 2 or 3 capsules of size 0, 1 or 2 in the normal or EL format administered once nightly.

In some embodiments of any of the above aspects the amantadine is administered as a pharmaceutically acceptable salt. In a more specific embodiment, the amantadine is administered as amantadine hydrochloride or amantadine sulfate.

In some embodiment of any of the above aspects, a total daily dose of 260 mg to 420 mg is administered as a once nightly formulation after 4 pm and/or within 4 hours of bedtime. In some embodiments, the once nightly dose of amantadine or pharmaceutically acceptable salt thereof administered exceeds 300 mg per day (e.g., 300 to 420 mg per day). The dose of the invention comprises 220 mg to 600 mg of amantadine or a pharmaceutically acceptable salt thereof. In various specific embodiments, the once daily or nightly dose of amantadine or pharmaceutically acceptable salt thereof may be 230 to 255 mg, 250 to 275 mg, 260 to 275 mg, 270 to 285 mg, 280 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, or 410 to 420 mg. Once daily or nightly doses of 50 to 75 mg, 70 to 95 mg, 90 to 115 mg, 110 to 135 mg, 130 to 155 mg, 150 to 175 mg, 170 to 195 mg, 190 to 215 mg, 210 to 235 mg are also disclosed. In some preferred embodiments, the once daily or nightly dose of amantadine or pharmaceutically acceptable salt thereof is 260 mg to 360 mg, 300 to 360 mg, 330 to 350 mg or 340 mg, 430 to 445 mg or 440 to 455 mg.

In some embodiments of any of the above aspects, the once daily or nightly composition of amantadine or pharmaceutically acceptable salt thereof comprises from about 260 mg, 265 mg, 270 mg, 275 mg, 280 mg, 285 mg, 290 mg, 295 mg, or 300 mg of amantadine, or a pharmaceutically acceptable salt thereof to about 305 mg, 310 mg, 315 mg, 320 mg, 325 mg, 330 mg, 335 mg, 340 mg, 345 mg, 350 mg, 355 mg, 360 mg, 365 mg, 370 mg, 375 mg, 380 mg, 385 mg, 390 mg, 395 mg, 400 mg, 405 mg, 410 mg, 415 mg, or 420 mg of amantadine, or a pharmaceutically acceptable salt thereof.

In specific embodiments described herein (e.g., when the formulation has a median Tmax of 8 to 18 hours as determined from a single dose, fasted human PK study), a subject's entire daily dose of amantadine is administered once, during a period of less than about four, three, two or one hours before bedtime (i.e., after 4 pm and/or the time at which the subject wishes to go to sleep for the night).

In some embodiments described herein (e.g., when the formulation has a median Tmax of 5 to 11 hours as determined from a single dose, fasted human PK study), a subjects entire daily dose of amantadine may be administered once in the morning (e.g., after waking and not later than 12 pm).

In some embodiments of any of the above aspects, administration of the composition to an MS patient results in a significant reduction in symptoms associated with the disease or condition. In some specific embodiments, administration of the composition results in about 5%, 10%, 15%, 20%, 25%, 30%, 35%, or 40% reduction in MS symptoms or motor fluctuations. In further specific embodiments, the reduction in MS symptoms or motor fluctuations is measured on a numerical scale used by or accepted by the FDA or other regulatory agencies to evaluate the effectiveness of and to approve for licensure drugs for the treatment of MS symptoms or motor fluctuations.

Some embodiments described herein provide a dose for use in a method of improving Clinician's Global Impression without increasing sleep disturbances in a patient comprising administering to said patient once nightly, 0 to 4 hours before bed time a composition comprising 260 to 420 mg amantadine, or a pharmaceutically acceptable salt thereof (e.g., amantadine hydrochloride), and at least one release modifying excipient. In some such methods, the dose of amantadine, or pharmaceutically acceptable salt thereof, is from 300 to 360 mg per day, particularly 330 to 350 mg per day, and in particular 340 mg per day.

Some embodiments described herein provide a method of improving Clinician's Global Impression without increasing sleep disturbances in a patient comprising administering to said patient once daily, a composition comprising 260 to 420 mg amantadine, or a pharmaceutically acceptable salt thereof (e.g., amantadine hydrochloride), and at least one release modifying excipient. In some such methods, the dose of amantadine, or pharmaceutically acceptable salt thereof, is from 300 to 360 mg per day, particularly 330 to 350 mg per day, and in particular 340 mg per day. In some such methods, the C-ave-day is 1.2 to 1.7 times the C-ave-night; in preferred methods the C-ave-day is measured between the hours of 9 am to 5 pm and the C-ave-night is measured between the hours of 10 pm to 6 am. In some such methods, administration of a single dose of the composition to a cohort of human healthy volunteer subjects in a fasted state provides an average maximum plasma concentration (Cmax) of 1.4 to 2.8 ng/ml per mg of amantadine or an AUC_{0-inf} (Area under the concentration-curve curve from t = 0 to t = infinity) of 46 to 56 ng^{∗}h/mL per mg of amantadine or both. In some such methods, the daily oral administration of a dose of the composition to a cohort of human subjects provides a steady state plasma concentration profile characterized by at least one of: (i) a mean Cmax of 2.2 to 2.9 ng/ml per mg of amantadine, (ii) a mean Cmin of 1.4 to 1.7 ng/ml per mg of amantadine, and (iii) a mean AUC₀₋₂₄ of 46 to 56 ng^{∗}h/mL per mg of amantadine; in more specific methods, all three criteria of (i), (ii) and (iii) are met.

In some embodiments of any of the above aspects, administration of the composition to patients results in a significant improvement in clinicians overall impression. In some specific embodiments, administration of the composition results in about a 0.5, 1.0, 1.5, 2.0, 2.5 or 3.0 point improvement in clinicians overall impression using a 7 point scale (or proportionate changes using a different scale). In further specific embodiments, the improvement in clinicians overall impression is measured on a numeric scale that is used by or accepted by the FDA or other regulatory agencies to evaluate the effectiveness of and to approve for licensure drugs indicated for patients. In further specific embodiments, the scale used in measuring the improvement in clinicians overall impression could be the Clinicians Global Impression of Change Rating Scale (CGIC). In other specific embodiments, the improvement in clinicians overall impression is measured relative to placebo in a controlled clinical trial. In other embodiments, the improvement in clinicians overall impression is measured relative to baseline in a controlled clinical trial.

The subject of the invention has multiple sclerosis. In one embodiment of the invention, a patient with a gait abnormality is administered a composition comprising amantadine and at least one excipient. In certain embodiments, an immediate release formulation of amantadine or a pharmaceutically acceptable salt thereof is administered to a patient with multiple sclerosis or a patient after a stroke once or twice per day to provide an improvement in gait. In some of these embodiments, the daily dose of amantadine or a pharmaceutically acceptable salt thereof is 100 mg to 300 mg.

In certain embodiments, at least one of said excipients in the composition to be administered is a release modifying excipient.

In certain embodiments, the composition to be administered comprises an extended release form of amantadine or a pharmaceutically acceptable salt thereof. In some of these embodiments, the extended release form of amantadine or a pharmaceutically acceptable salt thereof is administered orally, once daily to provide an improvement in gait in a patient with a gait abnormality. In some of these embodiments, the gait abnormality is associated with multiple sclerosis, in the patient. In certain embodiments, the daily dose of amantadine or a pharmaceutically acceptable salt thereof is 220 mg to 600 mg, 220 mg to 445 mg, 240 mg to 445 mg, 240 mg to 420 mg, 240 mg to 340 mg. In some embodiments the daily dose of amantadine or a pharmaceutically acceptable salt thereof is 260 mg to 420 mg.

In certain embodiments, the patient is also administered orally one or more second agents selected from the group consisting of 4-aminopyridine, baclofen, dextromethorphan, dimethyl fumarate, fingolimod, methylphenidate, and teriflunomide, tetrabenazine, and tizanidine, including pharmaceutically acceptable salts thereof. In certain embodiments, the second agent(s) is provided separately from the amantadine composition. In certain embodiments, the second agent(s) is provided in the same composition as the amantadine or pharmaceutically acceptable salt thereof. In certain embodiments, the second agent(s) is provided in a controlled release form. In some embodiments, the total daily dose of the second agent(s) is 25%, 50%, 75%, 100% of the total daily dose provided in the approved product label for the second agent(s). In some embodiments, the second agent is administered separately, sequentially, or simultaneously with the amantadine or pharmaceutically acceptable salt thereof.

In certain embodiments, the daily administration of amantadine to patients with walking deficits provides an improvement (vs. placebo) in at least one of the following measures: TUG (timed up and go), T25FW (timed 25 foot walk test), or 6MWT (six minute walk test), or 2 minute walk test, or MSWS-12 (twelve item multiple sclerosis walking scale).

In some embodiments of the disclosure, a method of improving gait in a human subject is provided, said method comprising orally administering to said patient once daily in the morning or nightly, a dose comprising (i) 50 mg to 600 mg of a drug selected from the group consisting of amantadine and pharmaceutically acceptable salts thereof and (ii) at least one excipient (e.g., wherein the dose includes a composition comprising components (i) and (ii)). In some embodiments of the invention, the composition may comprise 100 mg to 450 mg of amantadine, or a pharmaceutically acceptable salt thereof. In some embodiments of the invention, the composition may comprise 130 - 210 mg of amantadine, or a pharmaceutically acceptable salt thereof. In some embodiments, a dosage form containing the composition comprises 50 to 75 mg, 70 to 95 mg, 90 to 115 mg, 110 to 135 mg, 130 to 155 mg, 150 to 175 mg, 170 to 195 mg, 190 to 215 mg, 210 to 235 mg, 230 to 255 mg, 250 to 275 mg, 270 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, 410 to 425 mg, 420 to 435 mg, 430 to 445 mg or 440 to 455 mg of amantadine, or a pharmaceutically acceptable salt thereof. In some embodiments, the composition comprises about 110, 120, 130, 140, 150, 160 170, 180, 190, 210, 220 or 340 mg amantadine, or a pharmaceutically acceptable salt thereof. In some embodiments, the composition comprises 110 mg amantadine hydrochloride. In some embodiments, the composition comprises 130 mg amantadine hydrochloride. In some embodiments, the composition comprises 170 mg amantadine hydrochloride. In some embodiments, the composition comprises 210 mg amantadine hydrochloride. In the invention, the human subject has multiple sclerosis. In some embodiments, the dose additionally comprises one or more drugs selected from the group consisting of 4-aminopyridine, baclofen, dextromethorphan, dimethyl fumarate, fingolimod, methylphenidate, and teriflunomide, tetrabenizine, and tizanidine. In some embodiments, the at least one excipient is a release modifying excipient. In some embodiments, at least one of said excipients (or the at least one excipient) modifies the release of the amantadine or pharmaceutically acceptable salt thereof to provide an extended release form, and wherein administration of the dose provides a) a Tmax for amantadine of 5 to 18 hours, b) a Cmax of 1.0 to 2.8 ng/ml per mg amantadine, and c) an AUC_{0-inf} of 40 to 75 ng^{∗}hr/ml per mg amantadine as measured in a single dose human pharmacokinetic study. In some embodiments, such dosing of amantadine results in peak plasma amantadine concentrations occurring in the morning or afternoon, and very low plasma concentrations in the night. In some embodiments, the drug is provided in an extended release form. In additional specific embodiments, peak amantadine plasma concentration is achieved between 5, 6, 7, 8, 9, 10, 11 or 12 hours to about 9, 10, 11, 12, 13, 14, 15, 16, 17, 18, 19, 20, 21, 22, 23 or 24 hours after administration of a single dose of the composition (e.g., 8 hours to about 18 hours after administration). In some embodiments described herein the amantadine composition is administered to a patient from 0 to 4 hours prior to bedtime. In some embodiments, the amantadine composition is administered to a patient from 0 to 3, 0 to 2, or 0 to 1 hours prior to bedtime. In some embodiments, the amantadine composition is administered to a patient from 0 to 240 minutes, from 0 to 180 minutes, e.g., from 0 to 120 minutes, from 0 to 60 minutes, from 0 to 45 minutes, from 0 to 30 minutes, from 0 to 15 minutes or from 0 to 10 minutes prior to bedtime. In some embodiments, the amantadine composition is administered to a patient from 60 to 240 minutes, from 60 to 180 minutes, from 60 to 120 minutes or from 60 to 90 minutes prior to bedtime. In some embodiments described herein the amantadine composition is administered to a patient in the morning; in more specific embodiments, the amantadine composition is administered to said patient after said patient wakes but before 3, 4, 5 or 6 hours after waking. In some embodiments described herein the amantadine composition is administered to a patient after the patient wakes in the morning and before 12 pm. In some embodiments of any of the above aspects, administration of the composition to a MS disease subjects results in a significant reduction in MS disease gait impairment. In a specific embodiment, administration of the composition results in at least about 5%, 10%, 15%, 20%, 25%, 30%, 35%, or 40%, 45%, 50%, 55%, or 60% reduction in MS disease gait impairment. In further specific embodiments, gait (or gait impairment) is measured on a numeric scale that is used by or accepted by the FDA or other regulatory agencies to evaluate the effectiveness of and to approve for licensure drugs for the treatment of gait impairment. In some embodiments, the once daily administration of the amantadine composition (or combination comprising amantadine) provides an improvement in a hypokinetic movement disorder such as gait impairment. The improvement may be determined by methods known in the art such as timed up and go (TUG), timed 25 foot walk test (T25FW), or six minute timed walk test (6MTW). In some embodiments, the once daily or once nightly composition is administered as one, two, three or four unit dosage forms in unequally or, preferably, equally divided units. In some more specific embodiments, the composition is administered as two or three unit dosage forms each comprising 85 to 175 mg amantadine, or a pharmaceutically acceptable salt thereof. In some embodiments, the composition is administered as two unit dosage forms each comprising 100 to 175 mg amantadine, or a pharmaceutically acceptable salt thereof. In some embodiments, the composition is administered as two or three unit dosage forms of unequal, or preferably equal, dosage, each comprising 85 to 250 mg amantadine, or a pharmaceutically acceptable salt thereof. In some more specific embodiments, the composition is administered as two unit dosage forms each comprising 150 to 180 mg amantadine, or a pharmaceutically acceptable salt thereof.

In some embodiments of the disclosure, a method of improving walking in a human subject is provided, said method comprising orally administering to said patient once daily or nightly, a dose comprising (i) 50 mg to 600 mg of a drug selected from the group consisting of amantadine and pharmaceutically acceptable salts thereof and (ii) at least one excipient (e.g., wherein the dose includes a composition comprising components (i) and (ii)). In some such embodiments, the methods treat the disorders and diseases defined herein. In some embodiments, the composition may comprise 100 mg to 450 mg of amantadine, or a pharmaceutically acceptable salt thereof. In some embodiments, the composition may comprise 130 - 210 mg of amantadine, or a pharmaceutically acceptable salt thereof. In various specific embodiments, a dosage form containing the composition comprises 50 to 75 mg, 70 to 95 mg, 90 to 115 mg, 110 to 135 mg, 130 to 155 mg, 150 to 175 mg, 170 to 195 mg, 190 to 215 mg, 210 to 235 mg, 230 to 255 mg, 250 to 275 mg, 270 to 295 mg, 290 to 305 mg, 300 to 315 mg, 310 to 325 mg, 320 to 335 mg, 330 to 345 mg, 340 to 355 mg, 350 to 365 mg, 360 to 375 mg, 370 to 385 mg, 380 to 395 mg, 390 to 405 mg, 400 to 415 mg, 410 to 425 mg, 420 to 435 mg, 430 to 445 mg or 440 to 455 mg of amantadine, or a pharmaceutically acceptable salt thereof. In some embodiments, the composition comprises about 110, 120, 130, 140, 150, 160 170, 180, 190, 210, 220 or 340 mg amantadine, or a pharmaceutically acceptable salt thereof. In some embodiments, the composition comprises 110 mg amantadine hydrochloride. In some embodiments, the composition comprises 130 mg amantadine hydrochloride. In some embodiments, the composition comprises 170 mg amantadine hydrochloride. In some embodiments, the composition comprises 210 mg amantadine hydrochloride. In some embodiments, the human subject has multiple sclerosis. In some embodiments, the dose additionally comprises one or more drugs selected from the group consisting of 4-aminopyridine, baclofen, dextromethorphan, dimethyl fumarate, fingolimod, methylphenidate, and teriflunomide, tetrabenizine, and tizanidine. In some embodiments, the at least one excipient is a release modifying excipient. In some embodiments, at least one of said excipients (or the at least one excipient) modifies the release of the amantadine or pharmaceutically acceptable salt thereof to provide an extended release form, and wherein administration of the dose provides a) a Tmax for amantadine of 5 to 18 hours, b) a Cmax of 1.0 to 2.8 ng/ml per mg amantadine, and c) an AUC_{0-inf} of 40 to 75 ng^{∗}hr/ml per mg amantadine as measured in a single dose human pharmacokinetic study. In some embodiments, such dosing of amantadine results in peak plasma amantadine concentrations occurring in the morning or afternoon, and very low plasma concentrations in the night. In some embodiments, the drug is provided in an extended release form. In additional specific embodiments, peak amantadine plasma concentration is achieved between 5, 6, 7, 8, 9, 10, 11 or 12 hours to about 9, 10, 11, 12, 13, 14, 15, 16, 17, 18, 19, 20, 21, 22, 23 or 24 hours after administration of a single dose of the composition (e.g., 8 hours to about 18 hours after administration). In some embodiments, peak amantadine plasma concentration is achieved between 5 and 9 hours, between 5 and 10 hours, between 5 and 11 hours, between 5 and 12 hours, between 5 and 13 hours, between 5 and 14 hours, between 5 and 16 hours, between 5 and 18 hours, between 5 and 20 hours, between 5 and 22 hours, between 5 and 24 hours, between 6 and 9 hours, between 6 and 10 hours, between 6 and 11 hours, between 6 and 12 hours, between 6 and 13 hours, between 6 and 14 hours, between 6 and 16 hours, between 6 and 18 hours, between 6 and 20 hours, between 6 and 22 hours, between 6 and 24 hours, after a single dose of the composition, between 7 and 9 hours, between 7 and 10 hours, between 7 and 11 hours, between 7 and 12 hours, between 7 and 13 hours, between 7 and 14 hours, between 7 and 16 hours, between 7 and 18 hours, between 7 and 20 hours, between 7 and 22 hours, between 7 and 24 hours, between 8 and 9 hours, between 8 and 10 hours, between 8 and 11 hours, between 8 and 12 hours, between 8 and 13 hours, between 8 and 14 hours, between 8 and 16 hours, between 8 and 18 hours, between 8 and 20 hours, between 8 and 22 hours, between 8 and 24 hours, between 9 and 10 hours, between 9 and 11 hours, between 9 and 12 hours, between 9 and 13 hours, between 9 and 14 hours, between 9 and 16 hours, between 9 and 18 hours, between 9 and 20 hours, between 9 and 22 hours, between 9 and 24 hours, between 10 and 11 hours, between 10 and 12 hours, between 10 and 13 hours, between 10 and 14 hours, between 10 and 16 hours, between 10 and 18 hours, between 10 and 20 hours, between 10 and 22 hours, between 10 and 24 hours, between 12 and 13 hours, between 12 and 14 hours, between 12 and 16 hours, between 12 and 18 hours, between 12 and 20 hours, between 12 and 22 hours, or between 12 and 24 hours, after administration of a single dose of the composition. In some embodiments described herein the amantadine composition is administered to a patient from 0 to 4 hours prior to bedtime. In some embodiments, the amantadine composition is administered to a patient from 0 to 3, 0 to 2, or 0 to 1 hours prior to bedtime. In some embodiments, the amantadine composition is administered to a patient from 0 to 240 minutes, from 0 to 180 minutes, e.g., from 0 to 120 minutes, from 0 to 60 minutes, from 0 to 45 minutes, from 0 to 30 minutes, from 0 to 15 minutes or from 0 to 10 minutes prior to bedtime. In some embodiments, the amantadine composition is administered to a patient from 60 to 240 minutes, from 60 to 180 minutes, from 60 to 120 minutes or from 60 to 90 minutes prior to bedtime. In some embodiments, walking speed or ability is evaluated via at least one of the following or a combination there of: Timed 25-Foot Walking test (T25FW), Timed Up and Go (TUG), 2 minute walk test and/or, Twelve Item Multiple Sclerosis Walking Scale (MSWS-12). In some embodiments, walking in the subject is significantly improved. In some embodiments, MS symptoms are improved. In some embodiments T25FW, TUG, 2 minute walk and/or MSWS-12 is significantly improved (relative to placebo). In some embodiments, administration of the composition to a MS disease subject results in a significant reduction in MS disease walking impairment. In a specific embodiment, administration of the composition results in at least about 5%, 10%, 15%, 20%, 25%, 30%, 35%, or 40%, 45%, 50%, 55%, or 60% reduction in MS disease walking impairment. In a specific embodiment, administration of the composition results in about 5%, 10%, 15%, 20%, 25%, 30%, 35%, or 40%, 45%, 50%, 55%, or 60% reduction in MS disease walking impairment. In further specific embodiments, the reduction in walking impairment is measured on a numeric scale that is used by or accepted by the FDA or other regulatory agencies to evaluate the effectiveness of and to approve for licensure drugs for the treatment of walking impairment. In some embodiments, the once daily or once nightly composition is administered as one, two, three or four unit dosage forms in unequally or, preferably, equally divided units. In some more specific embodiments, the composition is administered as two or three unit dosage forms each comprising 85 to 175 mg amantadine, or a pharmaceutically acceptable salt thereof. In some embodiments, the composition is administered as two unit dosage forms each comprising 100 to 175 mg amantadine, or a pharmaceutically acceptable salt thereof. In some embodiments, the composition is administered as two or three unit dosage forms of unequal, or preferably equal, dosage, each comprising 85 to 250 mg amantadine, or a pharmaceutically acceptable salt thereof. In some more specific embodiments, the composition is administered as two unit dosage forms each comprising 150 to 180 mg amantadine, or a pharmaceutically acceptable salt thereof.

### Extended Release Formulations

Extended release amantadine compositions suitable for use in the invention can be made using a variety of extended release technologies, such as those described in the patent publications referenced in the above background section. In some embodiments, the invention is a pellet in capsule dosage form. In some embodiments, the pellets comprise a pellet core, which is coated with at least one drug layer and at least one extended release coating layer. In some embodiments, the pellets are coated with at least one drug layer, an intermediate layer such as a seal coat and an extended release coating layer. In some embodiments, the pellet, the drug layer or both comprise one or more binders.

In some embodiments, the dosage unit comprises a plurality of coated pellets. In some embodiments, the pellets have a diameter of for example 300 to 1700 microns, in some cases 500 to 1200 microns. The pellets will comprise, for example, inert substrates, such as sugar spheres, microcrystalline cellulose (MCC) spheres, starch pellets. In some embodiments, pellets can be prepared by other processes such as pelletization, extrusion, spheronization, etc., or combinations thereof. The core pellets will comprise of amantadine hydrochloride and pharmaceutically acceptable excipients.

### Coated Pellets

The pellet cores are coated with the active ingredient, e.g., amantadine or a pharmaceutically acceptable salt and/or polymorph thereof. In some embodiments, in addition to the active ingredient, the pellets also comprise one or more binders, such as for example hydroxypropyl methyl cellulose, copovidone, povidone, hydroxypropyl cellulose, hydroxyethyl cellulose, methyl cellulose, carboxymethyl cellulose, etc. In some embodiments, the pellets also contain one or more additional excipients, such as anti-tack agents (e.g., talc, magnesium stearate, etc.)

In some embodiments, the pellets cores are coated with a drug layer comprising active ingredient, and optionally one or more binders, anti-tack agents and/or solvents by conventional coating techniques such as fluidized bed coating, pan coating.

### Intermediate Layer Coating

In some embodiments, the pellets are coated with an intermediate layer, such as a seal coat. In some embodiments, the seal coat is adapted to prevent ingredients in the extended release coating from interacting with ingredients in the pellet core, to prevent migration of the ingredients in the pellet core from diffusing out of the pellet core into the extended release layer, etc. As described herein, the seal coat of the present invention can comprise one or more film forming polymers including but not limited to hydroxypropylmethyl cellulose (HPMC), copovidone, povidone, polyvinyl pyrrolidone, hydroxypropyl cellulose, hydroxyethyl cellulose, methyl cellulose, carboxymethyl cellulose or any combination thereof and the like.

The seal coat can further comprise other additives like plasticizers, such as, propylene glycol, triacetin, polyethylene glycol, tributyl citrate and optionally anti-tacking agents, such as, magnesium stearate, calcium silicate, magnesium silicate, and colloidal silicon dioxide or talc.

Apart from plasticizers and anti-tacking agents as mentioned above, the seal coat can optionally contain buffers, colorants, opacifiers, surfactants or bases, which are known to those skilled in the art.

Seal coating can be applied to the core using conventional coating techniques such as fluidized bed coating, pan coating, etc. In some embodiments, the drug coated pellets cores are coated with a seal coat layer that optionally comprises one or more binders, anti-tack agents and/or solvents by fluidized bed coating or pan coating.

### Binders

In some embodiments, the pellet cores, the intermediate coating layer, or both may comprise one or more binders (e.g., film forming polymers). Suitable binders for use herein include, e.g.,: alginic acid and salts thereof; cellulose derivatives such as carboxymethylcellulose, methylcellulose (e.g., Methocel®), hydroxypropylmethylcellulose, hydroxyethylcellulose, hydroxypropylcellulose (e.g., Klucel®), ethylcellulose (e.g., Ethocel®), and microcrystalline cellulose (e.g., Avicel®); microcrystalline dextrose; amylose; magnesium aluminum silicate; polysaccharide acids; bentonites; gelatin; polyvinylpyrrolidone/vinyl acetate copolymer; crospovidone; povidone; starch; pregelatinized starch; tragacanth, dextrin, a sugar, such as sucrose (e.g., Dipac®), glucose, dextrose, molasses, mannitol, sorbitol, xylitol (e.g., Xylitab®), and lactose; a natural or synthetic gum such as acacia, tragacanth, ghatti gum, mucilage of isapol husks, polyvinylpyrrolidone (e.g., Polyvidone® CL, Kollidon® CL, Polyplasdone® XL-10), larch arabogalactan, Veegum®, polyethylene glycol, waxes, sodium alginate, and the like.

### Extended Release Coating

The pellets are coated with an extended release coating. The extended release coating is adapted to delay release of the drug from the coated drug cores for a period of time after introduction of the dosage form into the use environment. In some embodiments, the extended release coating includes one or more pH-dependent or non-pH-dependent extended release excipients. Examples of non-pH dependent extended release polymers include ethyl cellulose, hydroxypropylmethyl cellulose, hydroxyethyl cellulose, hydroxypropyl cellulose, carboxymethyl cellulose, copolymer of ethyl acrylate, methyl methacrylate (e.g., Eudragit RS), etc. Examples of pH dependent extended release excipients include methacrylic acid copolymers, hydroxypropylmethyl cellulose acetate succinate, hydroxypropylmethyl cellulose phthalate, and cellulose acetate phthalate, etc. The extended release coating may also include a pore former, such as povidone, polyethylene glycol, hydroxypropyl cellulose, hydroxypropylmethyl cellulose, etc., sugars such as sucrose, mannitol, lactose, and salts, such as sodium chloride, sodium citrate, etc., a plasticizer, such as acetylated citrated esters, acetylated glycerides, castor oil, citrate esters, dibutylsebacate, glyceryl monostearate, diethyl phthalate, glycerol, medium chain triglycerides, propylene glycol, polyethylene glycol. The extended release coating may also include one or more additional excipients, such as lubricants (e.g., magnesium stearate, talc, etc.).

Extended release coating can be applied using conventional coating techniques such as fluidized bed coating, pan coating, etc. The drug coated pellets cores, which optionally comprise a seal coat, are coated with the extended release coating by fluidized bed coating.

### Extended Release Excipients (Coating Polymers)

As described herein, exemplary extended release excipients include, but are not limited to, insoluble plastics, hydrophilic polymers, and fatty compounds. Plastic matrices include, but are not limited to, methyl acrylate-methyl methacrylate, polyvinyl chloride, and polyethylene. Hydrophilic polymers include, but are not limited to, cellulosic polymers such as methyl and ethyl cellulose, hydroxyalkyl celluloses such as hydroxypropyl cellulose, hydroxypropylmethyl cellulose, sodium carboxymethyl cellulose, and cross-linked acrylic acid polymers like Carbopol® 934, polyethylene oxides and mixtures thereof. Fatty compounds include, but are not limited to, various waxes such as carnauba wax and glyceryl tristearate and wax-type substances including hydrogenated castor oil or hydrogenated vegetable oil, or mixtures thereof.

In certain embodiments, the plastic material can be a pharmaceutically acceptable acrylic polymer, including but not limited to, acrylic acid and methacrylic acid copolymers, methyl methacrylate, methyl methacrylate copolymers, ethoxyethyl methacrylates, cyanoethyl methacrylate, aminoalkyl methacrylate copolymer, poly(acrylic acid), poly(methacrylic acid), methacrylic acid alkylamine copolymer poly(methyl methacrylate), poly(methacrylic acid)(anhydride), polymethacrylate, polyacrylamide, poly(methacrylic acid anhydride), and glycidyl methacrylate copolymers.

In certain other embodiments, the acrylic polymer is comprised of one or more ammonio methacrylate copolymers. Ammonio methacrylate copolymers are well known in the art, and are described in NF XVII as fully polymerized copolymers of acrylic and methacrylic acid esters with a low content of quaternary ammonium groups.

In still other embodiments, the acrylic polymer is an acrylic resin lacquer such as that which is commercially available from Rohm Pharma under the trade name Eudragit®. In further embodiments, the acrylic polymer comprises a mixture of two acrylic resin lacquers commercially available from Rohm Pharma under the trade names Eudragit® RL30D and Eudragit® RS30D, respectively. Eudragit® RL30D and Eudragit® RS30D are copolymers of acrylic and methacrylic esters with a low content of quaternary ammonium groups, the molar ratio of ammonium groups to the remaining neutral (meth)acrylic esters being 1:20 in Eudragit RL30D and 1:40 in Eudragit® RS30D. The mean molecular weight is about 150,000. Eudragit® S-100 and Eudragit® L-100 are also suitable for use herein. The code designations RL (high permeability) and RS (low permeability) refer to the permeability properties of these agents. Eudragit® RL/RS mixtures are insoluble in water and in digestive fluids. However, multiparticulate systems formed to include the same are swellable and permeable in aqueous solutions and digestive fluids.

The polymers described above such as Eudragit® RL/RS may be mixed together in any desired ratio in order to ultimately obtain an extended release formulation having a desirable dissolution profile. One skilled in the art will recognize that other acrylic polymers may also be used, such as, for example, Eudragit® L.

### Pore Formers

In some embodiments, the extended release coating includes a pore former. Pore formers suitable for use in the extended release coating can be organic or inorganic agents, and include materials that can be dissolved, extracted or leached from the coating in the environment of use. Examples of pore formers include but are not limited to organic compounds such as mono-, oligo-, and polysaccharides including sucrose, glucose, fructose, mannitol, mannose, galactose, lactose, sorbitol, pullulan, dextran; polymers soluble in the environment of use such as water-soluble hydrophilic polymers, such as povidone, crospovidone, polyethylene glycol, hydroxypropyl cellulose, hydroxypropylmethyl cellulose, hydroxyalkyl celluloses, carboxyalkyl celluloses, cellulose ethers, acrylic resins, polyvinylpyrrolidone, cross-linked polyvinylpyrrolidone, polyethylene oxide, carbowaxes, Carbopol®, and the like, diols, polyols, polyhydric alcohols, polyalkylene glycols, polyethylene glycols, polypropylene glycols, or block polymers thereof, polyglycols, poly(α-Ω) alkylenediols; inorganic compounds such as alkali metal salts, lithium carbonate, sodium chloride, sodium bromide, potassium chloride, potassium sulfate, potassium phosphate, sodium acetate, sodium citrate, suitable calcium salts, and the like. In certain embodiments, plasticizers can also be used as a pore former.

### Capsules

The extended release pellets may be introduced into a suitable capsule by using an encapsulator equipped with pellet dosing chamber. The capsule sizes may be 00, 0, 0EL, 1, 1EL, 2, 2EL, 3, 4 or 5. A particularly preferred composition that provides ideal pharmacokinetic properties and plasma concentration profiles is a pellet-in-capsule composition that comprises a plurality of pellets, typically having a diameter of about 500 µm to 1.2 mm, and preferably about 700 µm to 1000 µm, where each pellet comprises a core comprising amantadine and a binder, and an extended release coating surrounding the core that extends release of the amantadine so as to provide the desired pharmacokinetic properties and amantadine plasma concentration profiles described above.

In some embodiments, the pellets in the pellet-in-capsule are in a size 0 or smaller, preferably a size 1 or smaller capsule. Mean pellet diameters in some embodiments may be in a range of 500 µm to 1200 µm, e.g., from 500 µm to 1100 µm, from 500 µm to 1000 µm, from 500 µm to 900 µm, from 500 µm to 800 µm, from 500 µm to 700 µm, from 600 µm to 1100 µm, from 600 µm to 1000 µm, from 600 µm to 900 µm, from 600 µm to 800 µm, from 600 µm to 700 µm, from 700 µm to 1100 µm, from 700 µm to 1000 µm, from 700 µm to 900 µm, or from 700 µm to 800 µm. In some embodiments the mean particle diameters are, ±10%, e.g.,: 500 µm, 550 µm, 600 µm, 650 µm, 700 µm, 750 µm, 800 µm, 850 µm, 900 µm, 950 µm, 1000 µm, 1050 µm, 1100 µm, 1150 µm or 1200 µm.

One preferred composition of the invention is a pellet-in-capsule composition wherein each pellet comprises a core that comprises a core seed with a mixture of amantadine and a binder coated onto the core seed, and an extended release coating surrounding the core comprising ethyl cellulose, a pore forming agent such as hydroxypropyl methyl cellulose or povidone, and a plasticizer. In some embodiments, the pellets may further comprise a seal coating between the pellet core and the extended release coating. The pellets are formulated using methods known in the art, such as those described in Example 1 below. In a specific embodiment, based on the combined weight of the pellet core and extended release coating, the amantadine is present in amounts from 20-80 wt%, 45-70 wt%, 40-50 wt%, 45-55 wt%, 50-60 wt%, 55-65 wt%, 60-70 wt%, 65-75 wt%, 70-80 wt%, or 40 to 60 wt%, the binder, which is preferably hydroxypropyl methyl cellulose, copovidone, or mixtures thereof, is present in amounts from 1 to 25 wt%, the core seed, preferably a sugar sphere (nonpareil) or microcrystalline cellulose seed (e.g., Celphere®), is present in amounts from 8 to 25 wt%, the ethyl cellulose is present in amounts from 10 to 20 wt%, the pore forming agent, preferably povidone, is present in amounts from 1 to 4 wt%, and the plasticizer is present in amounts from 1 to 4 wt%. In another specific embodiment, based on the combined weight of the pellet core and extended release coating, the amantadine is present in amounts from 50 to 70 wt%, the binder, which is preferably hydroxypropyl methyl cellulose, copovidone, or mixtures thereof, is present in amounts from 1 to 25 wt%, the core seed, preferably a sugar sphere (nonpareil) or microcrystalline cellulose seed (e.g., Celphere®), is present in amounts from 5 to 15 wt%, the ethyl cellulose is present in amounts from 1 to 15 wt%, the pore forming agent, preferably povidone, is present in amounts from 0.25 to 4 wt%, and the plasticizer is present in amounts from 0.25 to 4 wt%.

Additional embodiments of the invention are illustrated in the Table 1, below, entitled "Various Amantadine ER Capsule Size 1 Formulations". By means of methods and compositions described herein, formulations can be made that achieve the desired dissolution characteristics and target pharmacokinetic profiles described herein. More specifically, therapeutically effective doses of amantadine can be administered once nightly in no more than two size 1 (or smaller, e.g., size 2 or 3) capsules using the manufacturing methods and compositions that have been described herein to achieve these results. In particular, higher drug loading can be achieved using compositions and manufacturing methods described herein. In some embodiments, higher drug loading may be achieved, with the required dissolution profile, using smaller core pellet sizes and concomitantly increased drug layering on smaller cores, but with no change in the extended release coat. In some embodiments, using alternative manufacturing approaches described herein, e.g., extrusion and spheronization, even higher drug loads can be achieved to realize the desired dissolution profile, enabling high amantadine drug loads with suitable pharmacokinetic profiles, resulting in compositions that are therapeutically more effective, and at least as well tolerated, and can be filled in relatively small sized capsules (e.g., size 1, 2 or 3), enabling ease of administration to patients.

**Table 1: Various Amantadine ER Capsule Size 1 Formulations**

| | | | | | | |
|---|---|---|---|---|---|---|
| | | | | | | |

| **AMT Strength (mg)** | **Manufacture Method** | **Inert Core Pellet Size (mm)** | **Active Drug % w/w** | **Extended Release Coating % w/w** | **Bulk Density (g/cm³)** | **% Fill in Size 1 Capsule** |
|---|---|---|---|---|---|---|
| 85 mg | Fluid bed coating | 0.3-0.5 | 40-50% | 10-30% | 0.6-1.0 | 60-70% |
| 110 mg | Fluid bed coating | 0.3-0.5 | 40-50% | 10-30% | 0.6-1.0 | 60-70% |
| 140 mg | Fluid bed coating | 0.3-0.5 | 45-50% | 10-30% | 0.6-1.0 | 80-90% |
| 150 mg | Fluid bed coating | 0.3-0.5 | 50-55% | 10-30% | 0.6-1.0 | 80-90% |
| 170 mg | Fluid bed coating | 0.2-0.3 | 50-55% | 10-30% | 0.6-1.0 | 80-90% |
| 170 mg | Extrusion spheronization, pan or fluidized bed coating | N/A | 55-75% | 10-30% | 0.6-1.0 | 65-75% |
| 190 mg | Extrusion spheronization, pan or fluidized bed coating | N/A | 55-75% | 10-30% | 0.6-1.0 | 75-85% |
| 210 mg | Extrusion spheronization, pan or fluidized bed coating | N/A | 55-75% | 10-30% | 0.6-1.0 | 80-90% |
| 230 mg | Extrusion spheronization, pan or fluidized bed coating | N/A | 55-75% | 10-30% | 0.6-1.0 | 85-95% |

In some embodiment, the amantadine, or a pharmaceutically acceptable salt thereof, is present in amounts from 20 to 80 wt % (based on the combined weight of the pellet core and extended release coating), with a bulk density of 0.3 to 1.2 g/cm³. In some embodiments, the amantadine or pharmaceutically acceptable salt thereof is present in amounts from 20 to 77.5 wt %, from 20 to 75 wt %, from 20 to 72.5 wt %, from 20 to 70 wt %, from 20 to 67.5 wt %, from 20 to 65 wt %, from 20 to 62.5 wt %, from 20 to 60 wt %, from 20 to 57.5 wt %, from 20 to 55 wt %, from 20 to 52.5 wt %, from 20 to 50 wt %, from 20 to 47.5 wt %, from 20 to 45 wt %, from 20 to 42.5 wt %, from 20 to 40 wt %, from 20 to 37.5 wt %, from 20 to 35 wt %, from 20 to 32.5 wt %, from 20 to 30 wt %, from 30 to 80 wt %, from 30 to 77.5 wt %, from 30 to 75 wt %, from 30 to 72.5 wt %, from 30 to 70 wt %, from 30 to 67.5 wt %, from 30 to 65 wt %, from 30 to 62.5 wt %, from 30 to 60 wt %, from 30 to 57.5 wt %, from 30 to 55 wt %, from 30 to 52.5 wt %, from 30 to 50 wt %, from 30 to 47.5 wt %, from 30 to 45 wt %, from 30 to 42.5 wt %, from 30 to 40 wt %, from 40 to 80 wt %, from 40 to 77.5 wt %, from 40 to 75 wt %, from 40 to 72.5 wt %, from 40 to 70 wt %, from 40 to 67.5 wt %, from 40 to 65 wt %, from 40 to 62.5 wt %, from 40 to 60 wt %, from 40 to 57.5 wt %, from 40 to 55 wt %, from 40 to 52.5 wt %, from 40 to 50 wt %, from 40 to 47.5 wt %, from 40 to 45 wt %, from 50 to 80 wt %, from 50 to 77.5 wt %, from 50 to 75 wt %, from 50 to 72.5 wt %, from 50 to 70 wt %, from 50 to 67.5 wt %, from 50 to 65 wt %, from 50 to 62.5 wt %, from 50 to 60 wt %, from 50 to 57.5 wt %, from 50 to 55 wt %, from 60 to 80 wt %, from 60 to 77.5 wt %, from 60 to 75 wt %, from 60 to 72.5 wt %, from 60 to 70 wt %, from 60 to 67.5 wt %, from 60 to 65 wt %. In some embodiments, the bulk density is 0.3 to 1.2 g/cm³, 0.3 to 1.15 g/cm³, 0.3 to 1.1 g/cm³, 0.3 to 1.05 g/cm³, 0.3 to 1.0 g/cm³, 0.3 to 0.9 g/cm³, 0.3 to 0.8 g/cm³, 0.3 to 0.7 g/cm³, 0.3 to 0.6 g/cm³, 0.3 to 0.5 g/cm³, 0.3 to 0.4 g/cm³, 0.4 to 1.2 g/cm³, 0.4 to 1.15 g/cm³, 0.4 to 1.1 g/cm³, 0.4 to 1.05 g/cm³, 0.4 to 1.0 g/cm³, 0.4 to 0.9 g/cm³, 0.4 to 0.8 g/cm³, 0.4 to 0.7 g/cm³, 0.4 to 0.6 g/cm³, 0.4 to 0.5 g/cm³, 0.5 to 1.2 g/cm³, 0.5 to 1.15 g/cm³, 0.5 to 1.1 g/cm³, 0.5 to 1.05 g/cm³, 0.5 to 1.0 g/cm³, 0.5 to 0.9 g/cm³, 0.5 to 0.8 g/cm³, 0.5 to 0.7 g/cm³, 0.5 to 0.6 g/cm³, 0.6 to 1.2 g/cm³, 0.6 to 1.15 g/cm³, 0.6 to 1.1 g/cm³, 0.6 to 1.05 g/cm³, 0.6 to 1.0 g/cm³, 0.6 to 0.9 g/cm³, 0.6 to 0.8 g/cm³, 0.6 to 0.7 g/cm³, 0.7 to 1.2 g/cm³, 0.7 to 1.15 g/cm³, 0.7 to 1.1 g/cm³, 0.7 to 1.05 g/cm³, 0.7 to 1.0 g/cm³, 0.7 to 0.9 g/cm³, 0.7 to 0.8 g/cm³, 0.5 to 1.2 g/cm³, 0.8 to 1.15 g/cm³, 0.8 to 1.1 g/cm³, 0.8 to 1.05 g/cm³, 0.8 to 1.0 g/cm³, 0.8 to 0.9 g/cm³, 0.9 to 1.2 g/cm³, 0.9 to 1.15 g/cm³, 0.9 to 1.1 g/cm³, 0.9 to 1.05 g/cm³, or 0.9 to 1.0 g/cm³. In some embodiments, the composition is in a dosage unit comprising a pellet in capsule formulation, wherein the capsule size is size 00, size 0, size 1, size 2 or size 3. In some preferred embodiments, the dosage unit includes pellets containing from 50 to 250 mg of amantadine in a size 0, 1, 2 or 3 capsule. In some embodiments, the dosage unit includes pellets containing from 100 to 250 mg, e.g., 100 to 200 mg of amantadine in a size 0, 1, 2 or 3 capsule, preferably a size 1, 2 or 3 capsule. In some embodiments, the dosage unit comprises about 110, 120, 130, 140, 150, 160 170, 180, 190, 210, or 220 mg amantadine, or a pharmaceutically acceptable salt thereof. In some embodiments, the dosage unit comprises 110 mg amantadine hydrochloride. In some embodiments, the dosage unit comprises 130 mg amantadine hydrochloride. In some embodiments, the dosage unit comprises 170 mg amantadine hydrochloride. In some embodiments, the dosage unit comprises 210 mg amantadine hydrochloride.

Suitable plasticizers include medium chain triglycerides, diethyl phthalate, citrate esters, polyethylene glycol, glycerol, acetylated glycerides, castor oil, and the like. The pellets are filled into capsules to provide the desired strength of amantadine. An advantage of this composition is it provides the desired release properties that make the composition suitable for administration during said period before bedtime. A further advantage is that the extended release coating is sufficiently durable so that the capsule can be opened and the pellets sprinkled onto food for administration to patients who have difficulty swallowing pills, without adversely affecting the release properties of the composition. When the composition is administered by sprinkling onto food, it is preferred to use a soft food such as applesauce or chocolate pudding, which is consumed within 30 minutes, and preferably within 15 minutes. A yet further advantage of the above-described composition is that it has very good batch-to-batch reproducibility and shelf-life stability.

In additional embodiments, 110 mg to 210 mg of ER amantadine in a size 1 capsule of the composition of the invention has an *in vitro* dissolution profile of amantadine of not more than 25% at 2 hours, 55-85% at 6 hours, and at least 80% at 12 hours, as measured using a USP Apparatus II (Paddles) at 50 rpm with 500 ml water at 37° C as the dissolution medium. More preferably, the *in vitro* dissolution is further characterized by release of amantadine of not more than 10 % at 1 hour, 30-50 % at 4 hours, and at least 90% at 12 hours.

In some embodiments, the composition has an in vitro dissolution profile of amantadine which shows at least one of (i) not more than 25% dissolution at 2 hours, (ii) not more than 25-55% dissolution at 6 hours, and (iii) at least 80% dissolution at 12 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

In some embodiments of any of the above aspects the composition has an in vitro dissolution profile of amantadine which shows at least one of (i) not more than 20% dissolution at 1 hour, (ii) about 25-45% dissolution at 2 hours, (iii) not more than 50-80% dissolution at 4 hours, and (iii) at least 80% dissolution at 8 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

In some embodiments of any of the above aspects the composition has an in vitro dissolution profile of amantadine which shows at least one of (i) about 35-55% dissolution at 2 hours, (ii) not more than 60-80% dissolution at 4 hours, and (iii) at least 90% dissolution at 8 hours, using a USP Apparatus II (Paddles) at 50 rpm with 500 ml water at 37° C as the dissolution medium. In some embodiments two of criteria (i), (ii) and (iii) are met. In some embodiments, all three of criteria (i), (ii) and (iii) are met.

A preferred pellet-in-capsule composition of the invention, in addition to having the above *in vitro* dissolution properties and any of the above-described pharmacokinetic properties (e.g., *in vivo* release profile, Tmax, Cmax/Cmin ratio, etc.) that make the composition suitable for administration in said period before bedtime. The composition is further characterized by providing a Cmax of 1.6-2.4 ng/ml per mg of amantadine and an AUC_{0-inf} of 40-75 ng^{∗}h/mL per mg of amantadine after oral administration of a single dose of the capsule to a human subject in a fasted state. A preferred pellet-in-capsule composition is further characterized by a steady state plasma concentration in which once nightly oral administration of the capsule to a human subject provides a Cmax of 2.4 to 4.2 ng/ml per mg of amantadine, a Cmin of 1.1 to 2.6 ng/ml per mg of amantadine, and an AUC₀₋₂₄ of 48-73 ng^{∗}h/mL per mg of amantadine.

The above-described pellet-in-capsule compositions may be provided at a strength suitable for amantadine therapy. Typical strengths range from at least about 50 mg to about 250 mg. In a specific embodiment, the capsule strength is 70 mg, 80 mg, 85 mg, 90 mg, 110 mg, 120 mg, 125 mg, 130 mg, 140 mg, 150 mg, 160 mg, 160mg, 170 mg, 180 mg, 190 mg, 210 mg, and 220 mg, that provides a single dose AUC_{0-inf} per mg that is equivalent to a 100 mg tablet of an immediate release formulation of amantadine HC1 (e.g., Symmetrel®, or other FDA Orange Book reference listed drug). One, two, or three, of such capsules can be administered to a subject in the period before bedtime. In a preferred embodiment, between 220 mg and 650 mg of amantadine is adminstered using 2 capsules of a suitable ER formulations once nightly.

### Other Extended Release Dosage Forms

The person of skill in the art will recognize that other embodiments of extended release compositions may be envisioned, in addition to the capsule formulation described above. Such other embodiments include extended release solid dosage forms, such as tablets, capsules, gel caps, powders, pellets, beadlets, etc. Included in such extended release compositions are those that have the release characteristics and *in vivo* pharmacokinetic profile to be employed in the invention. In some embodiments, the person skilled in the art may employ, with appropriate adjustment of design characteristics to achieve the necessary pharmacokinetic profile described herein, the extended release technology described in U.S. Patent No. 5,358,721, to Guittard et al., or U.S. Patent No. 6,217,905, to Edgren et al., or U.S. Patent No. 8,574,626, to Vergez et al., each of which disclose an oral osmotic dosage form of amantadine. In other embodiments, the person of skill in the art may employ, again with appropriate adjustment of design characteristics, the technology described in U.S. Patent No. 6,194,000, to Smith et al., Patent No. 8,741,343 or U.S. Patent Appl. Publication Nos. US 2006/0252788, US 2006/0189694, US 2006/0142398, US 2008/0227743 US2011/0189273, US2015/0045446, US2015/0051292 or US2014/0242163, all to Went et al., each of which disclose the administration of an NMDA receptor antagonist, such as amantadine, optionally in controlled release form.

Some embodiments herein provide a dose of the invention for use in a method of once nightly administering amantadine (or a pharmaceutically acceptable salt thereof, such as amantadine hydrochloride) to a subject in need thereof, said method comprising orally administering an extended release (ER) composition comprising amantadine, or a pharmaceutically acceptable salt thereof, less than four hours before bedtime (and/or after 4 pm). In some embodiments, administration occurs less than four hours before bedtime. In some embodiments, the method improves clinician global impression, and does so without inducing or increasing sleep disturbances in the patient. In some embodiments, the composition is added to food prior to administration. In some embodiments, there is no increase in plasma concentration of amantadine for at least one hour after the administration. In some embodiments, there is no increase in plasma concentration of amantadine for at least two hours after the administration. In some embodiments, the amantadine has a single dose Tmax of 9 to 18 hours, and/or a steady state Tmax of 7 to 13 hours. In some embodiments, the amantadine has a single dose Tmax of 12 to 18 hours after administration, and/or a steady state Tmax of 8 to 12 hours. In some embodiments, the amantadine has a single dose Tmax of 12 to 16 hours after administration, and/or a steady state Tmax of 9 to 12 hours. In some embodiments, a once nightly oral administration of the composition to a human subject provides a steady state plasma concentration profile characterized by a concentration increase of amantadine of less than 25% at three hours after the administration. In some embodiments, the PK curve has a Cmax/Cmin ratio of 1.3 to 3.0. In some embodiments, the ratio of C-ave-day/C-ave night at steady state is 1.4 to 1.7. In some embodiments, the average amantadine plasma concentration during the day (C-ave-day) at steady state is 500-2000 ng/ml. In some embodiments, the amantadine is amantadine hydrochloride or amantadine sulfate. In some embodiments, the composition comprises 260 to 420 mg of amantadine, or a pharmaceutically acceptable salt thereof. In some embodiments, the composition is administered as two, or three or four unit dosage forms each comprising 85 to 175 mg amantadine, or a pharmaceutically acceptable salt thereof. In some embodiments, the composition is administered as two unit dosage forms each comprising 130 to 210 mg of extended release amantadine, or a pharmaceutically acceptable salt thereof. In some embodiments, the composition is within a capsule of capsule size #1. In some embodiments, the composition comprises 260 mg to 340 mg of amantadine or pharmaceutically acceptable salt thereof. In some embodiments, the composition comprises 340 mg of amantadine or pharmaceutically acceptable salt thereof. In some embodiments, the composition comprises 170 mg amantadine hydrochloride. In some embodiments, oral administration of a single dose of the composition to a human subject in a fasted state provides a maximum plasma concentration (Cmax) of 1.1 to 1.7 ng/ml per mg of amantadine, and an AUC_{0-inf} of 46 to 56 ng^{∗}h/mL per mg of amantadine. In some embodiments, once nightly oral administration of a dose of the composition to a human subject provides a steady state plasma concentration profile characterized by: (a) a Cmax of 2.0 to 3.1 ng/ml per mg of amantadine; (b) a Cmin of 1.3 to 2.0 ng/ml per mg of amantadine, and (c) an AUC₀₋₂₄ of 46 to 56 ng^{∗}h/mL per mg of amantadine. In some embodiments, the steady state plasma concentration profile is further characterized by: (d) no increase in plasma concentration of amantadine for at least one hour after the administration; and (e) a Cmax/Cmin ratio of 1.3 to 3.0. In some embodiments, the steady state plasma concentration profile is further characterized by: (f) no increase in concentration of amantadine for at least two hours after the administration; and (g) a Cmax/Cmin ratio of 1.4 to 2.6.

In some embodiments, the composition has an *in vitro* dissolution profile of amantadine of not more than 25% at 2 hours, 55-85% at 6 hours, and at least 80% at 12 hours, using a USP Apparatus II (Paddles) at 50 rpm with 500 ml water at 37° C as the dissolution medium. In some embodiments, the composition has an *in vitro* dissolution profile of amantadine of not more than 25% at 2 hours, 25-55% at 6 hours, and at least 80% at 12 hours, using a USP Apparatus II (Paddles) at 50 rpm with 500 ml water at 37° C as the dissolution medium. In some embodiments, the composition has an *in vitro* dissolution profile of amantadine of not more than 20% at 1 hour, 25-45% at 2 hours, 50-80% at 4 hours, and at least 80% at 8 hours, using a USP Apparatus II (Paddles) at 50 rpm with 500 ml water at 37° C as the dissolution medium. In some embodiments, the *in vitro* dissolution profile of amantadine is further characterized by release of amantadine of not more than 10 % at 1 hour, 30-50 % at 4 hours, and at least 90% at 12 hours. In some embodiments, the *in vitro* dissolution properties facilitate the pharmacokinetic properties described herein. In some embodiments, the composition has an AUC profile after administration of a single dose of the composition characterized by: a fractional AUC from 0 to 4 hours that is less than 5% of AUCo-inf; a fractional AUC from 0 to 8 hours that is about 5 to 15% of AUC_{0-inf}; a fractional AUC from 0 to 12 hours that is about 10 to 40% of AUCo-inf; a fractional AUC from 0 to 18 hours that is about 25 to 60% of AUCo-inf; and a fractional AUC from 0 to 24 hours that is about 40 to 75% of AUC_{0-inf}. In some embodiments, the composition has an AUC profile after once nightly dosing of the composition at steady state conditions characterized by: a fractional AUC from 0 to 4 hours that is about 2 to 25% of AUC₀₋₂₄; a fractional AUC from 0 to 8 hours that is about 15 to 50% of AUC₀₋₂₄; a fractional AUC from 0 to 12 hours that is about 30 to 70% of AUC₀₋₂₄: and a fractional AUC from 0 to 18 hours that is about 60 to 95% of AUC₀₋₂₄.

Some embodiments herein provide a pharmaceutical composition for any of the methods described herein, wherein said composition is for oral administration and comprises at least one capsule for oral administration, said capsule comprising a plurality of pellets, each pellet comprising: (a) a pellet core comprising amantadine, or a pharmaceutically acceptable salt thereof, and (b) an extended release coating surrounding the pellet core. In some embodiments the composition comprises two of said capsules. In some embodiments, the extended release coating comprises ethyl cellulose, at least one of povidone and hydroxypropyl methyl cellulose, and a plasticizer. In some embodiments, the pellet core comprises amantadine, or a pharmaceutically acceptable salt thereof, and a binder coated onto a core seed. In some embodiments, based on the combined weight of the pellet core and extended release coating, the amantadine is present in amounts from 40 to 60 wt %, the binder is present in amounts from 8 to 25 wt %, the core seed is present in amounts from 1 to 25 wt %, the ethyl cellulose is present in amounts from 10 to 20 wt %, the povidone is present in amounts from 1 to 4 wt %, and the plasticizer is present in amounts from 1 to 4 wt %. In some embodiments, the composition further comprises a seal coating between the pellet core and the extended release coating. In some embodiments, the pellet core comprises a binder selected from the group consisting of hydroxypropyl methyl cellulose, copovidone, and mixtures thereof. In some embodiments, the plasticizer is selected from the group consisting of medium chain triglycerides, diethyl phthalate, citrate esters, polyethylene glycol, glycerol, acetylated glycerides and castor oil.

Some embodiments herein provide a dose of the invention for use in a method of administering amantadine, or a pharmaceutically acceptable salt thereof, to a human subject in need thereof, said method comprising orally administering a pharmaceutical composition comprising amantadine in at least one capsule for oral administration (e.g., one capsule), said capsule comprising a plurality of pellets, each pellet comprising: (a) a pellet core comprising amantadine, or a pharmaceutically acceptable salt thereof, and (b) an extended release coating surrounding the pellet core. In some embodiments the composition comprises two of said capsules. In some embodiments, the extended release coating comprises ethyl cellulose, at least one of povidone and hydroxypropyl methyl cellulose, and a plasticizer. In some embodiments, the pellet core comprises amantadine, or a pharmaceutically acceptable salt thereof, and a binder coated onto a core seed. In some embodiments, based on the combined weight of the pellet core and extended release coating, the amantadine is present in amounts from 40 to 60 wt %, the binder is present in amounts from 8 to 25 wt %, the core seed is present in amounts from 1 to 25 wt %, the ethyl cellulose is present in amounts from 10 to 20 wt %, the povidone is present in amounts from 1 to 4 wt %, and the plasticizer is present in amounts from 1 to 4 wt %. In some embodiments, the composition further comprises a seal coating between the pellet core and the extended release coating. In some embodiments, the pellet core comprises a binder selected from the group consisting of hydroxypropyl methyl cellulose, copovidone, and mixtures thereof. In some embodiments, the plasticizer is selected from the group consisting of medium chain triglycerides, diethyl phthalate, citrate esters, polyethylene glycol, glycerol, acetylated glycerides and castor oil.

Some embodiments herein provide a pharmaceutical composition suitable for once daily oral administration to a patient in need thereof said composition comprising a therapeutically effective amount of amantadine or a pharmaceutically acceptable salt thereof in an extended release form which can be administered as not more than two size 0 or smaller capsules in a single daily administration. In some embodiments, the composition comprises 110-220 mg of amantadine or pharmaceutically acceptable salt thereof. In some embodiments, the composition has an *in vitro* dissolution profile of amantadine of not more than 25% at 2 hours, 40-80% at 6 hours, and at least 80% at 12 hours, using a USP Apparatus II (Paddles) at 50 rpm with 500 ml water at 37° C as the dissolution medium. In some embodiments, the composition comprises a plurality of pellets, each pellet comprising: (a) a pellet core comprising amantadine, or a pharmaceutically acceptable salt thereof, and (b) an extended release coating surrounding the pellet core. In some embodiments, the extended release coating comprises ethyl cellulose, at least one of povidone and hydroxypropyl methyl cellulose, and a plasticizer. In some embodiments, the pellet core comprises amantadine, or a pharmaceutically acceptable salt thereof, and a binder coated onto a core seed. In some embodiments, the composition comprises amantadine and, based on the combined weight of the pellet core and extended release coating, the amantadine is present in amounts from 40 to 70 wt %. In some embodiments, the pellet core comprises a core seed comprising sugar or microcrystalline cellulose that is between 100 and 500 microns in diameter. In some embodiments, the bulk density is between 0.5 and 1 gm/cm³. In some embodiments, the composition comprises a seal coating between the pellet core and the extended release coating. In some embodiments, the pellet core comprises a binder selected from the group consisting of hydroxypropyl methyl cellulose, copovidone, and mixtures thereof. In some embodiments, the plasticizer is selected from the group consisting of medium chain triglycerides, diethyl phthalate, citrate esters, polyethylene glycol, glycerol, acetylated glycerides and castor oil.

Some embodiments herein provide a dose of the invention for use in a method of reducing sleep disturbance in a human subject undergoing treatment with amantadine, said method comprising once nightly administering an extended release (ER) composition comprising amantadine, or a pharmaceutically acceptable salt thereof, less than four hours before bedtime (and/or after 4 pm). In some embodiments, the composition is added to food prior to administration. In some embodiments, there is no increase in plasma concentration of amantadine for at least one hour after the administration. In some embodiments, the composition is added to food prior to administration. In some embodiments, there is no increase in plasma concentration of amantadine for at least one hour after the administration. In some embodiments, there is no increase in plasma concentration of amantadine for at least two hours after the administration.

In some embodiments, the once daily administration of the amantadine composition (or combination comprising amantadine) provides an improvement in a hypokinetic movement disorder such as gait. The improvement may be determined by methods known in the art such as timed up and go (TUG), timed 25 foot walk test (T25FW), or six minute timed walk test (6MTW). In patients with multiple sclerosis, the amantadine composition or combination comprising amantadine provides an improvement in fatigue which may be determined by the fatigue scale motor cognitive (FSMC). In patients with depression, the amantadine composition or combination comprising amantadine provides an improvement in depression which may be determined by Beck's Depression Inventory-2 (BDI-2). In patients with multiple sclerosis, the amantadine composition or combination comprising amantadine provides an improvement in walking which may be determined by the Timed 25-Foot Walking test (T25FW), Timed Up and Go (TUG), 2 minute walk test and/or, Twelve Item Multiple Sclerosis Walking Scale (MSWS-12). In patients with multiple sclerosis, the amantadine composition or combination comprising amantadine provides an improvement in cognition which may be determined by the Symbol Digit Modalities Test (SDMT), California Verbal Learning Test second edition (CVLT-II) with delayed recall, Brief Visuospatial Memory Test Revised (BVMT-R) with delayed recall, and/or Brief International Cognitive Assessment for Multiple Sclerosis (BICAMS). For instance, in some embodiments the present invention can provide improvement as determined by the evaluation in example 10 and/or example 11.

The present invention may be better understood by reference to the following examples.

### Example 1: Amantadine Extended Release Coated Pellet Formulations

Amantadine HCl extended release coated pellet compositions designed for nighttime administration were prepared using the components and relative amounts shown in Table 2, below. For each composition, the drug coating solution was prepared by adding HPMC 5 cps and Copovidone to isopropyl alcohol with continuous stirring. Purified water was added to this dispersion and stirring continued until a clear solution is formed. Drug (Amantadine HC1) was then added to this binder solution and stirring continued until the drug was completely dissolved. Finally, talc was added and dispersed uniformly by stirring.

Celphere beads (screen sizes #35 to #50 i.e., 300 to 500 micron) were loaded into a Wurster coating unit. The drug coating dispersion was sprayed onto the beads followed by a period of drying. The resulting drug coated pellets were sieved to retain the fraction between screens #18 and #24 (approximately 700 µm to 1mm diameter).

The seal coating solution was prepared by adding HPMC 5 cps to isopropyl alcohol with continuous stirring. Purified water was added to this dispersion and stirring continued until a clear solution was formed. Talc was added and dispersed uniformly by stirring. The sieved drug coated pellets were loaded in a Wurster coating unit. The seal coating dispersion was sprayed over the drug coated pellets followed by a period of drying to remove the residual solvent and water in the pellets. The resulting seal coated pellets were sieved to retain the fraction between screens #18 and #24.

The ER coating solution was prepared by dissolving ethyl cellulose (viscosity 7 cps) in isopropyl alcohol and purified water and stirring until a clear solution was formed. Povidone K-90 was then dissolved in this clear solution followed by addition of plasticizer Miglyol 812N with continuous stirring to form a clear solution. The sieved seal coated pellets were loaded in a Wurster coating unit. The ER coating solution was sprayed over the seal coated pellets followed by a period of drying to affect the ER coat and remove the residual solvent and water in the pellets. After drying, magnesium stearate was spread on the top bed of the coated pellets in the annulus region followed by recirculation of the pellets in the Wurster unit to blend the magnesium stearate with the coated pellets. The resulting ER coated pellets were sieved to retain the fraction between screens #18 and #24.

The desired weight of the ER coated pellets containing the unit dose were filled into empty 1 hard gelatin capsule shell (size 1 for 60 - 140 mg strength) using an encapsulator equipped with pellet dosing chamber.

**Table 2: Composition of amantadine HCI ER capsules**

| **Component** | **Function** | **combined w/w of capsule** |
|---|---|---|
| **Pellet Core** | | |
| Amantadine Hydrochloride USP | Active | 40-50% |
| Microcrystalline cellulose spheres (Celphere®) | Core seeds | 10-15% |
| Hydroxypropyl methyl cellulose 5 cps USP | Binder | 10-15% |
| Copovidone | Binder | 1-5% |
| Talc USP | Anti-tack | 1-5% |
| Isopropyl alcohol | Solvent | _¹ |
| Water | Solvent | _¹ |

| **Seal Coating (optional)** | | |
|---|---|---|
| Hydroxypropyl methyl cellulose 3 cps USP | Coating polymer | 5-10% |
| Talc USP | Anti-tack | 0-5% |
| Isopropyl alcohol | Solvent | _¹ |
| Water | Solvent | _¹ |

| **Extended Release Coating** | | |
|---|---|---|
| Ethyl cellulose | Coating polymer | 10-20% |
| Povidone | Pore former | 1-5% |
| Medium chain triglycerides | Plasticizer | 1-5% |
| Isopropyl alcohol | Solvent | _¹ |
| Water | Solvent | _¹ |
| Magnesium Stearate NF | Lubricant | 0-1% |
| | | |
| Density of pellets | | 0.6 - 0.9 gm/cm³ |

| | | |
|---|---|---|
| NF = National Formulary ¹ Purified water and isopropyl alcohol are removed during processing. | | |

The in vitro dissolution of capsules prepared above was tested using a USP Apparatus II (Paddles) at 50 rpm with 500 ml water at 37° C as the dissolution medium. Capsules meeting desired dissolution specifications released not more than 25% of the drug in 2 hours, 40-80% in 6 hours, and at least 80% at 12 hours. In an exemplary dissolution profile, there was 0% drug release at 1 hour, 12% release at 2 hours, 43% release at 4 hours, 68% release at 6 hours, 83% release at 8 hours, 92% release at 10 hours, and 97% release at 12 hours. Capsules prepared in accordance with the above method exhibited good shelf-stability, and batch-to-batch reproducibility upon scale-up.

### Example 2: Amantadine extended release coated pellet formulation with higher drug loading

Amantadine HC1 extended release coated pellet compositions designed for nighttime administration are prepared using the components and relative amounts shown in Table 3 below and the manufacturing process described in Example 1.

The diameter of the inert cores is 200 - 300 microns. The diameter of the coated pellets is 600 - 1200 microns. The bulk density of the coated pellets is 0.7 - 1.2 g/cm³.

The desired weight of the ER coated pellets containing the unit dose are filled into an empty hard gelatin capsule shell (size 1 for 170 mg strength) using an encapsulator equipped with pellet dosing chamber.

**Table 3: Composition of amantadine HCl ER capsules**

| **Component** | **Function** | **combined w/w of capsule** |
|---|---|---|
| **Pellet Core** | | |
| Amantadine Hydrochloride USP | Active | 50-65% |
| Microcrystalline cellulose spheres (Celphere®) | Core seeds | 1-15% |
| Hydroxypropyl methyl cellulose USP | Binder | 5-25% |
| Copovidone | Binder | 1-5% |
| Talc USP | Anti-tack | 1-5% |
| Isopropyl alcohol | Solvent | _¹ |
| Water | Solvent | _¹ |

| **Seal Coating (optional)** | | |
|---|---|---|
| Hydroxypropyl methyl cellulose USP | Coating polymer | 0-10% |
| Talc USP | Anti-tack | 0-5% |
| Isopropyl alcohol | Solvent | _¹ |
| Water | Solvent | _¹ |

| **Extended Release Coating** | | |
|---|---|---|
| Ethyl cellulose | Coating polymer | 10-20% |
| Povidone | Pore former | 1-5% |
| Medium chain triglycerides | Plasticizer | 1-5% |
| Isopropyl alcohol | Solvent | _¹ |
| Water | Solvent | _¹ |
| Magnesium Stearate NF | Lubricant | 0-1% |

| | | |
|---|---|---|
| NF = National Formulary ¹ Purified water and isopropyl alcohol are removed during processing. | | |

The *in vitro* dissolution of capsules prepared above are tested using a USP Apparatus II (Paddles) at 50 rpm with 500 ml water at 37° C as the dissolution medium and release not more than 25% of the drug in 2 hours, 40-80% in 6 hours, and at least 80% at 12 hours.

### Example 3: Amantadine Extended Release Coated Pellet Formulations

Amantadine HC1 extended release coated pellet compositions suitable for nighttime administration were prepared using the components and relative amounts shown in Table 4 below and the manufacturing process described in Example 1.

**Table 4: Composition of amantadine HCI ER capsules**

| **Component** | **Function** | **combined w/w of capsule** |
|---|---|---|
| **Pellet Core** | | |
| Amantadine Hydrochloride USP | Active | 45.15% |
| Microcrystalline cellulose spheres (Celphere®) | Core seeds | 12.90% |
| Hydroxypropyl methyl cellulose USP | Binder/Coating polymer | 18.89% |
| Copovidone | Binder | 3.01% |
| Ethyl cellulose | Coating polymer | 13.53% |
| Povidone | Pore former | 1.84% |
| Medium chain triglycerides | Plasticizer | 1.62% |
| Talc USP | Anti-tack | 2.95% |
| Magnesium Stearate NF | Lubricant | 0.10% |
| | | |
| Isopropyl alcohol | Solvent | _¹ |
| Water | Solvent | _¹ |

| | | |
|---|---|---|
| NF = National Formulary ¹ Purified water and isopropyl alcohol are removed during processing. | | |

The desired weight of the ER coated pellets containing the unit dose was filled into empty #1 hard gelatin capsule shells (60, 140 mg strengths) using an encapsulator equipped with pellet dosing chamber. These dosage forms were used to provide the amantadine for the study described in Example 4 below according to the combinations in Table 5, as follows:

**Table 5: Capsules for Dosing**

| Dose for Study | 60 mg Capsules | 140 mg Capsules |
|---|---|---|
| 260 mg | 2 | 1 |
| 340 mg | 1 | 2 |
| 420 mg | 0 | 3 |

### Example 4: Pharmacokinetic measurement of a Formulation of Amantadine ER compared to IR amantadine

Objective: The primary objective of the study is to evaluate the pharmacokinetic profile, safety and tolerability of a prototype formulation of ER amantadine HCl (Formulation 1), relative to a 100 mg film-coated IR amantadine HCl tablet (SYMMETREL®) given as single doses to healthy adult subjects under fasting conditions.

Study design: This is a Phase 1, randomized, single dose, open-label, two-period, two-treatment crossover, fasting pharmacokinetic study in which single 340 mg doses of formulation 1 of Amantadine ER capsules is compared to single 100 mg doses of marketed amantadine IR tablets (SYMMETREL®).

Methods: Subjects are admitted to the unit for the first period of dosing within 21 days of study screening. There will be a 7 day washout between dosing in period 1 and 2. In each dosing period subjects will be dosed on the day after checking into the unit and discharged 72 hours post dose. A final follow up end of study will be conducted within 14 days of dosing in the second period.

After an overnight fast, the formulation is administered to the subjects while in a sitting position with 240 mL of water. Blood samples were collected at 0 (pre-dose), 1, 2, 3, 4, 6, 7, 8, 9, 10, 11, 12, 13, 14, 15, 16, 18, 24, 30, 36, 48, 60, 72 hours following each dose. Plasma samples are assayed for amantadine by a validated liquid chromatography/tandem mass spectroscopy (LC/MS/MS) method. Pharmacokinetic parameters are calculated using a non-compartmental analysis with WinNonlin software (version 5.3 or higher; Pharsight Corporation).

An analysis of variance (ANOVA) is performed on the natural logarithms of Cmax and AUC_{0-inf} determined from the data following a single dose of study drug using linear mixed effects model. The model includes sequence, period, and regimen as fixed effects and subject with sequence as a random effect. Ratio of ER to IR for both AUC (relative bioavailability for ER formulation) and Cmax is calculated. Adverse events are monitored throughout the study. Vital signs (pulse rate, blood pressure and body temperature), clinical laboratory measures (biochemistry, hematology, and urinalysis) and ECGs are collected at various times during the study.

Expected Results: A total of 20 subjects comprising healthy male and female adults participate in the study.

The PK results from this study demonstrate a reduced Cmax (on a dose proportionate basis) for the Amantadine ER relative to the IR form (about 1.1 to 1.7 ng/ml/mg amantadine for the ER form versus about 2.7 ng/ml/mg amantadine for the IR form). Also, the Tmax for the Amantadine ER is 9 to 18 hours vs about 4 hours for the IR form. Total amantadine exposure, as measured by AUC_{0-inf}, for the Amantadine ER formulation is 80 to 100 percent of SYMMETREL® on a dose adjusted basis.). Figure 1 shows a plot of estimated amantadine plasma concentrations per mg amantadine dosed versus scheduled time for the ER formulation. The high and low curves bracket the range of mean values predicted at various times after dosing. These results are summarized in Table 6, below.

**Table 6: Single Dose Pharmacokinetic Parameters of Amantadine ER (Formulation 1), as Compared to SYMMETREL® (Formulation IR)**

| **Parameter ^{a}** | **Amantadine ER Formulation A** | **SYMMETREL Formulation IR** |
|---|---|---|
| Cₘₐₓ (ng/mL)/mg amantadine | 1.1 to 1.7 | 2.0 to 3.5 |
| Tₘₐₓ (h) [range] | 12 to 18 | 2 to 6 |
| AUC_{0-inf} (ng^{∗}h/mL)/mg amantadine | 46 to 56 | 54 to 65 |

### Example 5: Steady State Plasma Amantadine Concentration (ng/mL) Following Once Daily Dosing of 260 mg, 340 mg and 420 mg doses of ER Amantadine HCl (Formulation 1).

The steady state plasma amantadine concentration were predicted for ER amantadine formulation 1 (260 mg, 340 mg and 420 mg) given once a day based on a model obtained using WINNONLIN from the observed data from a previous single dose study (Study 5103-C-101). The steady state predictions were done using the principles of superposition using the observed single dose data and linear kinetics was assumed to generate the profiles at various dose levels (260 mg, 340 mg and 420 mg). Figure 2 shows the profiles for ER amantadine formulation 1 (260 mg, 340 mg and 420 mg) given once a day.

### Example 6: Amantadine Extended Release Compositions

Amantadine HCl extended release coated pellet compositions suitable for nighttime administration were prepared from the ER coated pellets prepared as described in Example 1 and filled into empty hard gelatin capsule shells as described in Table 7, below.

**Table 7: Amantadine HCl ER capsules**

| Capsule Strength (mg Amantadine) | Capsule Size | ER Coated Pellets (mg) |
|---|---|---|
| 85 mg | 2 | 188.3 |
| 100 mg | 2 | 221.5 |
| 160 mg | 1el | 354.4 |
| 170 mg | 0 | 376.5 |
| 200 mg | 0el | 443.0 |

### Example 7: Amantadine Extended Release Coated Pellet Formulations

Amantadine HCl extended release coated pellet compositions suitable for once daily administration were prepared using the components and relative amounts shown in Table 8, below, and the manufacturing process described in Example 1.

The desired weight of the ER coated pellets containing the unit dose was filled into empty #1 hard gelatin capsule shell (100 mg strength) using an encapsulator equipped with pellet dosing chamber.

**Table 8: Composition of amantadine HCl ER capsules**

| **Component** | **Function** | **combined w/w of capsule** | | |
|---|---|---|---|---|
| | | **A** | **B** | **C** |
| **Pellet Core** | | | | |
| Amantadine Hydrochloride USP | Active | 50.15% | 47.94% | 45.15% |
| Microcrystalline cellulose spheres (Celphere®) | Core seeds | 14.33% | 13.70% | 12.90% |
| Hydroxypropyl methyl cellulose USP | Binder | 13.37% | 12.79% | 12.04% |
| Copovidone | Binder | 3.34% | 3.2% | 3.01% |
| Talc USP | Anti-tack | 2.51% | 2.4% | 2.26% |
| Isopropyl alcohol | Solvent | -¹ | -¹ | -¹ |
| Water | Solvent | -¹ | -¹ | -¹ |

| **Seal Coating (optional)** | | | | |
|---|---|---|---|---|
| Hydroxypropyl methyl cellulose USP | Coating polymer | 7.61% | 7.27% | 6.85% |
| Talc USP | Anti-tack | 0.76% | 0.73% | 0.69% |
| Isopropyl alcohol | Solvent | -¹ | -¹ | -¹ |
| Water | Solvent | -¹ | -¹ | -¹ |

| **Extended Release Coating** | | | | |
|---|---|---|---|---|
| Ethyl cellulose | Coating polymer | 6.23% | 9.46% | 13.53% |
| Povidone | Pore former | 0.85% | 1.29% | 1.84% |
| Medium chain triglycerides | Plasticizer | 0.75% | 1.13% | 1.62% |
| Isopropyl alcohol | Solvent | -¹ | -¹ | -¹ |
| Water | Solvent | -¹ | -¹ | -¹ |
| Magnesium Stearate NF | Lubricant | 0.1% | 0.1% | 0.1% |

| | | | | |
|---|---|---|---|---|
| NF = National Formulary ¹ Purified water and isopropyl alcohol are removed during processing. | | | | |

The *in vitro* dissolution of capsules prepared above were tested using a USP Apparatus II (Paddles) at 50 rpm with 500 ml water at 37° C as the dissolution medium. The results are shown in Figure 3.

### Example 8: Pharmacokinetic measurement of Formulations of Amantadine ER compared to IR amantadine

Objective: The primary objective of the study was to confirm the PK properties of extended release formulations in example 7, to determine the pharmacokinetic profiles, safety and tolerability of three prototype formulations of ER capsules of amantadine HCl described with different release properties in Example 7 relative to a 100 mg film-coated IR amantadine HCl tablet (SYMMETREL®) given as single doses to healthy adult subjects under fasting conditions.

Study design: This was a Phase 1, randomized, single dose, open-label, four-period, crossover, fasting pharmacokinetic study in which single 100 mg doses of three formulations of Amantadine ER capsules with different release properties were compared to single 100 mg doses of marketed amantadine IR tablets (SYMMETREL®). The three ER formulations differed in the amantadine release rates in vitro, as shown in Figure 3.

Methods: Subjects were admitted to the unit for the first period of dosing within 21 days of study screening. Subjects were dosed on the day after checking into the unit and discharged at 24 hours post dose. Subjects were asked to return after discharge for follow-up visits at 56 hours and 152 hours after dosing. Each dosing period was separated by at least 7 day washout.

After an overnight fast, the formulation was administered to the subjects after waking in the morning while in a sitting position with 240 mL of water. Blood samples were collected at 0 (pre-dose), 1, 2, 3, 4, 5, 6, 7, 8, 9, 10, 11, 12, 13, 14, 15, 16, 18, 24 (discharge), and 56 hours following each dose. Plasma samples were assayed for amantadine by a validated liquid chromatography/tandem mass spectroscopy (LC/MS/MS) method. Pharmacokinetic parameters were calculated using a non-compartmental analysis with WinNonlin software (version 4.1 or higher; Pharsight Corporation).

An analysis of variance (ANOVA) was performed on the natural logarithms of Cmax and AUC_{0-∞} determined from the data following a single dose of study drug using linear mixed effects model. The model included effects for subject, sequence, period, and regimen. The effects of sequence, period, and regimen were fixed, while the effect of subject was random. Ratio of ER to IR for both AUC (relative bioavailability for ER formulations) and Cmax was calculated. (Adverse events were monitored throughout the study. Vital signs (pulse rate, blood pressure and body temperature), clinical laboratory measures (biochemistry, hematology, and urinalysis) and ECGs were collected at various times during the study.

Results: A total of 20 subjects participated in the study. The mean age was 25.5 years old (range 20-38 years). The study consisted of 8 male (40%) and 12 female (60%) subjects with a mean body mass index (BMI) of 23.6 kg/m2 ± 2.85. The racial makeup was 100% Caucasian. Fifteen subjects received all 4 treatments.

The PK results from this study showed that all three of the Amantadine ER formulations reduced the rate of absorption, based on the reduced values of Cmax and increased Tmax, compared to SYMMETREL® (Table 9, Figures 4, 5). The IR formulation had the highest mean Cmax (277 ± 73.9 ng/mL) and shortest median Tmax (4 h) values. Formulations A, B, and C produced progressively lower Cmax and longer Tmax values. Cmax decreased from 204 ± 61.4 to 166 ± 34.8 to 149 ± 34.4 ng/mL, and median Tmax increased from 7.0, to 11.0, to 14.0 h for formulations A, B, and C, respectively. Total amantadine exposure, as measured by AUC_{0-∞}, was slightly lower in all three Amantadine ER formulations than SYMMETREL® but all three formulations had acceptable bioavailability (85-95%). Table 10 summarizes the Cₘₐₓ and AUC_{0-∞} ratios (ER/IR) of ER Formulations A, B and C relative to IR.

**Table 9: Single Dose Pharmacokinetic Parameters of Three Formulations of Amantadine ER (Formulation A, B, and C), as Compared to SYMMETREL® (Formulation IR)**

| **Parameter ^{a}** | **100 mg Formulation A (n=19)** | **100 mg Formulation B (n=17)** | **100 mg Formulation C (n=18)** | **100 mg Formulation IR (n=18)** |
|---|---|---|---|---|
| Cₘₐₓ (ng/mL) | 204±61 | 166 ± 35 | 149 ± 34 | 277 ± 74 |
| Tₘₐₓ (h) [range] | 7 [5-11] | 11 [5-15] | 14 [₉₋₁8] | 4 [2-6] |
| AU₀₋ₗₐₛₜ (ng^{∗}h_{/mL}) | 5064 ± 1573 | 5028 ± 2328 | 4525 ± 1268 | 5488 ± 1730 |
| AUC_{0-∞} (ng^{∗}h/mL) | 5545 ± ₁₉04 | 5724 ± 2369 | 5652 ± 2581 | 5907 ± 1907 |
| t_{1/2} (h) | 13.9 ± 3.0 | 16.3 ± 5.2 | 18.3 ± 7.5 | 12.3 ± 3.5 |

| | | | | |
|---|---|---|---|---|
| • ^{a} All parameters are reported as the mean ± standard deviation (SD), except Tₘₐₓ which is reported as a median value (min to max range) | | | | |

**Table 10: Ratio ER/IR for Cₘₐₓ and AUC_{0-∞}**

| **Comparison** | **Variable** | **ER/IR^{a}** |
|---|---|---|
| | | |
| A vs. IR | Cₘₐₓ (ng/mL) | 66.0% |
| | AUC_{0-∞} (ng^{∗}h/mL) | 85.3% |
| B vs. IR | Cₘₐₓ (ng/mL) | 60.9% |
| | AUC_{0-∞} (ng^{∗}h/mL) | 94.6% |
| C vs. IR | Cₘₐₓ (ng/mL) | 51.2% |
| | AUC_{0-∞} (ng^{∗}h/mL) | 88.5% |

| | | |
|---|---|---|
| • ^{a}Point estimate of the geometric mean ratio (ER/IR). | | |

### Example 9: Evaluation of Gait Parameters in an EAE Mouse Model of Multiple Sclerosis

The primary objective of this study was to determine the timing and extent of gait disturbances in MOG35-55 induced experimental autoimmune encephalomyelitis (EAE) in C57BL/6 mice, an animal model of multiple sclerosis, and the effect(s) of amantadine on said model. The primary endpoints were disease severity and changes in fine motor parameters as measured by kinematic gait analysis.

### STUDY MATERIALS

### 1. Test Animals

Female C57BL/6 mice, 10-12 weeks old, and weighing between 16-22 grams from Charles River (Germany) were used for this study. The mice were housed 4 per cage for at least 4 days prior to use and maintained on standard rodent chow and tap water in the animal facility.

### 2. Test Articles

Amantadine HCl (AMT) was manufactured by MOEHS CATALANA, S.L. (Barcelona, Spain).

Dalfampridine (4-Aminopyridine, DAL) was purchased by CRL from Sigma.

### EXPERIMENTAL PROCEDURES

### 1. Test Groups

Animals were assigned to treatment groups according to the study design in Table 11. Animals were allocated into treatment groups to ensure similar distribution of body weights across all groups (±10% difference in mean body weight between groups).

**Table 11: Study Design**

| **Group** | **MOG35-55 Treatment** | **Treatment Group** | **Vehicle Type** | **Dose (mg/kg/day)^{a}** | **Pump Conc. (mg/mL)** | **Terminal Time Point (Day)^{b}** | **N (per Group)** |
|---|---|---|---|---|---|---|---|
| **1** | **Sham (no MOG)** | **Vehicle** | **25 mM Sodium acetate pH 5.0 + 0.9% saline** | **0** | **0** | **27** | **10** |
| **2** | **MOG₃₅₋₅₅** | **Vehicle** | **25 mM Sodium acetate pH 5.0 + 0.9% saline** | **0** | **0** | **27** | **10** |
| **3** | **MOG₃₅₋₅₅** | **AMT** | **25 mM Sodium acetate pH 5.0 + 0.9% saline** | **60** | **200** | **27** | **10** |
| **4** | **MOG₃₅₋₅₅** | **DAL** | **Water** | **4** | **13.3** | **27** | **10** |

| | | | | | | | |
|---|---|---|---|---|---|---|---|
| ^{a}AMT and DAL doses were based on the mean animal body weight of each group. If an animal's body weight was >10% different from the mean animal body weight in its respective group, then that animal's pump concentration was adjusted to its body weight. ^{b}Alzet minipumps were implanted on Day 0. | | | | | | | |

### 2. Dose Selection

The selected dose of amantadine listed in Table 11 used was based on a previous Alzet osmotic minipump study in mice, in order to achieve steady-state plasma concentrations of approximately 1200 ng/mL.

The dose of dalfampridine listed in Table 11 was based on Göbel et al, to achieve steady-state plasma concentrations in mice of approximately 50 ng/mL.

### 3. Test Article Preparation, Administration, Analyses and Disposition

### 3.1. Preparation of Dosing Solutions

Sodium acetate buffer (pH 5.0) was prepared from a 25 mM solution of acetic acid and a 25 mM solution of sodium acetate in 0.9% sterile saline by adding the sodium acetate solution into the acetic acid solution until a pH of 5.0 is reached. The final solution was filtered over a 0.2 µm filter prior to use.

Amantadine HCl was weighed into sterile volumetric flasks and adjusted to volume with 25 mM sodium acetate pH 5.0 in 0.9% saline to produce the concentrations specified in Table 12. The pH was adjusted to pH 5.0 with 4M sodium acetate. Dalfampridine was weighed into sterile volumetric flasks and adjusted to volume with sterile water to produce the concentrations specified in Table 11. All solutions were filtered through a 0.2 µm filter under aseptic conditions.

### 3.2. Formulation Collection

Two aliquots of 100 µL each of each batch of compound formulation were collected after preparation. At termination (Day 27), after pump removal from the mouse, one aliquot was collected from each pump. The formulation samples were collected into glass vials and stored at -70 to -80°C until analysis at the completion of the study.

### 3.3. Alzet Minipump Preparation

Animals were implanted subcutaneously with Alzet #2004 osmotic minipumps (0.25 µL/h, 28 day pump) that had been primed with amantadine or dalfampridine at concentrations specified in Table 11. Prior to surgery, minipumps were primed by the following protocol. The minipumps were filled with the appropriate drug solution using a sterile needle and syringe. The osmotic caps were then placed onto the minipumps. The minipumps were then cleaned with an isopropanol-soaked tissue, allowed to dry and then put into a container of sterile 0.9% saline for 6-16 hours at 37°C to prime the pumps. Pumps from the same group were primed together in one container.

### 4. In-Life Procedures

### 4.1. Alzet Minipump Implantation Surgery

Animals were implanted with osmotic pumps containing AMT or DAL at concentrations specified in Table 11. Mice were anesthetized with 5% isoflurane after which isoflurane content in oxygen/nitrous oxygen was reduced to 1.5-2% for maintenance throughout the surgical procedure. Rectal temperature was maintained at 36.9±1.0 with a homeothermic heating blanket and rectal probe. Anesthetized mice were placed on the surgical table and the skin of the back was shaved and disinfected with povidone-iodine solution (Betadine, Leiras, 457028). Before incision, mice were given an analgesic (Temgesic, 1 mL/kg, s.c., RB Pharmaceuticals Ltd, 2014-07).

Thereafter, a small incision was made to expose the subcutaneous space for the Alzet minipump installation. The primed osmotic minipump was wiped with isopropanol. After the alcohol evaporated the pump was inserted into the pocket, with the delivery portal end first, thus minimizing interaction between delivered test compound and the healing of the incision. The skin was closed with 7-0 monofilament sutures and the mice were allowed to recover in single cages before returning them to their home cage (3-4 mice/cage). In addition, mice were given analgesic (Temgesic, 1 mL/kg, s.c., RB Pharmaceuticals Ltd, 2014-07) on day 0 (minimum 6 h post-surgery), day 1 (AM and PM) and day 2 (AM).

Implantation surgery and MOG inoculation was done concurrently on the same day (day 0).

### 4.2. Induction and Clinical Scoring of EAE

Mice were inoculated using commercially available ready to use inoculum (EK-0115, Hooke Laboratories, USA) containing 100 µg of MOG35-55, 200 µg heat inactivated of Mycobacterium tuberculosis in mineral oil in 100 µL of inoculum. Inoculation was done by giving each mouse 2 x 100 µl injections subcutaneously to lower and higher aspect of the back. Intraperitoneal injections of pertussis toxin (4 µg/mL) 100 µL each were given at inoculation and 24 hours after inoculation.

Clinical signs were scored daily by investigators blind to the treatment groups as described in Table 12.

**Table 12: Clinical Scoring of EAE**

| **Score** | **Manifestations** |
|---|---|
| **0.5** | **Partial tail weakness** |
| **1.0** | **Complete tail paralysis (all of tail dragged along)** |
| **1.5** | **Flaccid tail and abnormal gait** |
| **2** | **Flaccid tail and clear weakness of hind legs** |
| **2.5** | **Partial paralysis in one hind limb - (no movement preserved in affected limb).** |
| **3** | **Complete paralysis in both hind limbs** |
| **4** | **Complete paralysis in hind limbs and partial weakness in forelimbs** |
| **5** | **Complete paralysis in both forelimbs and hind limbs (Tetraplegia), moribund** |

### 4.3. Body Weights and General Clinical Observations

Body weights were measured on Day 0 (pre and post minipump implantation) and once daily thereafter. The time and weight will be recorded. General clinical observations and inspection of the implantation site of animals was conducted and recorded once daily. Gentle palpitation of the implantation site of the animals was conducted daily to prevent scar tissue from forming around the pump. Animals showing substantial signs of toxicity (i.e., prostrate, cold body temperature) and/or in moribund condition were euthanized by CO2 asphyxiation followed by decapitation prior to scheduled sacrifice.

Definitions of acceptable endpoints requiring euthanasia included: no spontaneous movements and inability to drink or eat in 24-h observation period, massive bleeding, spontaneous inflammation, missing anatomy, swelling or tumors larger than 20 mm, and inability to right itself in a 30 second period. Model (EAE) specific end-points justifying sacrifice include: clinical score at 4 (partial weakness in all limbs or hemiplegia) for more than 1 day without improvement (score below 4), righting reflex >30 seconds, and body weight drop over 25% from the baseline.

### 4.4. Kinematic Gait Analysis

On days 1, 5, 8, 11, 14, 17, 20, 23 and 26 all mice per group were be run in the MotoRater (TSE Systems, Hamburg, Germany) using the walking mode. The captured videos of each mouse were first converted to SimiMotion software to track the marked points of body (e.g., paws, hips, tail, etc.), and the movement of these different body points in relation to the ground were recorded in coordinates. Different gait patterns and fine motor movements were analyzed using a custom made automated analysis system. Kinematic endpoints are described in Table 13.

**Table 13: Kinematic Endpoints: Walking Mode**

| **Endpoint** | **Description** | **Unit** |
|---|---|---|
| Hind Limb Step Width between left and right fore paws mm | Distance between left and right hind paws | mm |
| Fore Limb Step Width Distance | Distance between left and right Fore paws | mm |
| Stride Time | Time duration between two consecutive paw placements of the same paw | s |
| Stride Distance | Step distance between two consecutive paw placements of the same paw | mm |
| Stride Speed | Distance the mouse walks per second | m/s |
| Stance Time | Time duration during which the paw is in contact with the surface | **s** |
| Swing Time | Time interval between two consecutive paw placements of the same paw in which the paw is not in contact with the surface | **s** |
| Inter-limb coordination | Homolateral, homologous and diagonal proportions of gait. | **U** |
| Tail tip and base height. | Relative to ground and tip height relative to base. | **mm** |

### 4.5. Terminal Procedures and Tissue Processing

At the termination time point (Day 27), all mice were deeply anesthetized with pentobarbital and blood samples were collected by cardiac puncture. Approximately 0.4-0.5 mL blood were collected into 500 µL plastic lavender K2EDTA anticoagulant tubes, and stored on wet ice until centrifugation. Blood samples were centrifuged (within 15 min of collection) at 3000 g for 10 min at 4°C. Signs of hemolysis (plasma is pink or red) was noted. The plasma was aliquoted into a single 1.5 mL Eppendorf tube per animal and placed into a storage box with separators and stored at -70 to -80oC until shipment. Additional tissues were not collected for this study.

### 7. BIOANALYTICAL ANALYSIS

Bioanalytical analysis of the plasma and formulations was performed by LC/MS/MS using established methods for amantadine and dalfampridine in order to determine the amantadine and dalfampridine concentrations, respectively.

### 8. DATA ANALYSIS

The plasma compound concentration at the terminal time point was tabulated for each animal by group, and the mean (± SD) concentration for each group was calculated.

Individual and mean (± SD) absolute body weights and body weight change from day 1 were tabulated and plotted as a function of time.

Clinical scores were graphed as mean scores (± SEM) as a function of time. Time of onset of disease and cumulative disease scores were graphed as individual values in a scatter graph and/or as mean (± SEM) in a bar graph.

Kinematic parameters (Table 13) were graphed as individual values in a scatter graph and/or as mean (± SEM) in a bar graph.

### 9. STATISTICAL ANALYSIS

All values were presented as mean ± standard deviation (SD) or standard error of mean (SEM), and differences were considered to be statistically significant at the p<0.05 level. Statistical analysis was performed using StatsDirect statistical software. Differences among means are analyzed by using 1-way-ANOVA followed by Dunnet's test (comparison to the control group).

Within-group comparison to the baseline is done by 2-way-ANOVA. Non-parametric data is analyzed with Kruskal-Wallis ANOVA or Friedman ANOVA, respectively.

### Results:

A total of 34 mice were used in the study, with 10 being used as SHAM-Vehicle controls, and 10 being used as Vehicle controls, 10 being treated with amantadine and 10 being treated with dalfampridine.

The results from this study show that amantadine treatment decreased symptom severity in a mouse model of MS. Figure 6 shows the progression of disease in the EAE mouse model for MS, with clinical scores increasing starting to increase between days 12-13, and peaking between days 18-20 in the EAE mice. Mice treated with both amantadine and dalfampridine show lower clinical scores than the untreated EAE mice, indicating that the drugs decreased symptoms in these groups compared to the untreated control. Neither group treated with drug showed a delay onset of disease in the EAE mouse model for MS.

A scatter plot of the cumulative clinical score (Day 18-27) for each group (Figure 7), both the group treated with amantadine, and the group treated with dalfampridine have reduced cumulative clinical scores compared to the untreated control.

Figure 8 shows expression of IBa-1 expression, a marker of microglia activation, in each of the assay groups. The amantadine-treated group showed significantly lower expression, indicating amantadine significantly reduces neuroinflammation in an animal model for MS.

Figure 9 provides a line graph showing the effect of disease progression on animal subjects and the impairment of their performance in the walk test. The untreated EAE control group drastically lost ability to complete the walk test after Day 14, with only 40% able to complete the test on Day 16. In comparison, 60% of the amantadine-treated group, and 50% of the dalfampridine-treated group, were able to complete the walking test on day 16. The Day 14 walking test provided the latest time point with most complete gait data.

Further analysis of the Gait study results indicated that amantadine improved walking speed at day 14. The amantadine-treated mice showed a lower time in the 60 cm Walk Time test (Figure 10A) as well as a faster mean walking speed (Figure 10B) than the untreated EAE control as well as the dalfampridine-treated group.

### Example 10: Evaluation of Amantadine for Treatment of Gait Disorders in Multiple Sclerosis Patients

Amantadine HCl extended release coated pellet compositions of Example 2 are compared to placebo in a double-blind, 2 arm study in subjects with MS with walking deficits as defined by EDSS (Expanded Disability Status Scale - Kurtzke 1983). All subjects receive a stable regimen of MS medications (excluding 4-aminopyridine) for at least 30 days prior to screening, and continue the same doses and regimen for the duration of the study. The subjects included in the study are randomized to one of two treatment groups: placebo or 340 mg amantadine in an extended release formulation. Study medications are administered once nightly at bedtime.

Subjects who are initially randomized to placebo receive placebo for the initial 1 week run-in period and throughout the 4 week randomized treatment period. Subjects who are initially randomized to active treatment receive placebo for the initial 1 week run-in period, followed by 170 mg amantadine extended release formulation for 1 week, followed by 340 mg amantadine extended release formulation for the remaining 3 week treatment period.

Adverse events are recorded beginning with the first dose of study drug and continue through the last study visit. Concomitant medications are recorded throughout the study.

Outcome measures:
A. Timed 25 foot walk test (T25FW - Kieseier and Pozzili 2012)
B. Timed up and go (TUG - Learmonth, Paul et al. 2012)
C. Six minute walk test (6MWT - Goldman, Marrie et al. 2008)
D. Fatigue Scale for Motor and Cognitive functions (FSMC - Penner Raselli et al., 2009)
E. Beck Depression Inventory-2 (BDI-2 - Smarr and Keefer 2011; Watson, Ford et al. 2014)

The expected result for the active treatment arm is an improvement in at least one of the aforementioned outcome measures. The T25FW, TUG, or 6MWT results for the active treatment arm are expected to be better than those for placebo.

### Example 11: Evaluation of Amantadine for Treatment of Walking Deficits in Multiple Sclerosis Patients

Amantadine HCl extended release coated pellet compositions described herein (e.g., those of Example 2) are administered to subjects with MS with walking deficits as defined by EDSS (Expanded Disability Status Scale - Kurtzke 1983). All subjects receive a stable regimen of MS medications (excluding 4-aminopyridine) for at least 30 days prior to screening, and continue the same doses and regimen for the duration of the study. The subjects are randomized to one of two treatment groups: placebo or 340 mg amantadine in an extended release formulation. Study medications are administered once nightly at bedtime.

Subjects initially randomized to placebo receive placebo for the initial 1 week run-in period and throughout the 4 week randomized treatment period. Subjects initially randomized to active treatment receive placebo for the initial 1 week run-in period, followed by 170 mg amantadine extended release formulation for 1 week, followed by 340 mg amantadine extended release formulation for the remaining 3 week treatment period.

Adverse events are recorded beginning with the first dose of study drug and continue through the last study visit. Concomitant medications are recorded throughout the study.

Walking speed or ability are evaluated via at least one of the following or a combination there of: Timed 25-Foot Walking test (T25FW), Timed Up and Go (TUG), 2 minute walk test, six minute timed walk test (6MTW), and/or, Twelve Item Multiple Sclerosis Walking Scale (MSWS-12). In some embodiments, walking in the subject is significantly improved. The result for the active treatment arm is an improvement in at least one of the aforementioned outcome measures. The T25FW, TUG, 2 minute walk test, 6MWT, or MSWS-12 results for the active treatment arm are better than those for placebo. MS symptoms improve. T25FW, TUG, 2 minute walk, 6MWT and/or MSWS-12 significantly improve relative to placebo.

## Claims

1. A dose comprising (i) 220 mg to 600 mg of amantadine or pharmaceutically acceptable salts thereof and (ii) at least one excipient, for use in a method of improving gait in a human subject with multiple sclerosis, the method comprising orally administering the dose to said subject once daily.

2. The dose for use according to claim 1, wherein the dose additionally comprises one or more drugs selected from the group consisting of 4-aminopyridine, baclofen, dextromethorphan, dimethyl fumarate, fingolimod, methylphenidate, teriflunomide, tetrabenizine, tizanidine, and pharmaceutically acceptable salts thereof.

3. The dose for use according to claim 2, wherein the one or more drugs are provided in an extended release form.

4. The dose for use according to any one of claims 1 to 3, wherein at least one excipient is a release modifying excipient.

5. The dose for use according to any one of claims 1 to 3, wherein at least one of said excipients modifies the release of the amantadine or pharmaceutically acceptable salt thereof to provide an extended release form, and wherein administration of the dose provides a) a Tmax for amantadine of 5 to 18 hours, b) a Cmax of 1.0 to 2.8 ng/ml per mg amantadine, and c) an AUC_{0-inf} of 40 to 75 ng^{∗}hr/ml per mg amantadine as measured in a single dose human pharmacokinetic study.

6. The dose for use according to any one of claims 1 to 5, wherein said amantadine or pharmaceutically acceptable salt thereof is provided in an extended release form.

7. The dose for use according to any one of claims 1 to 6, wherein said dose is administered in the morning and the composition provides a median Tmax of 5 to 9 hours as determined from a single dose, fasted, human pharmacokinetic study.

8. The dose for use according to any one of claims 1 to 6, wherein said dose is administered 0 to 4 hours before bedtime and the composition provides a median Tmax of 10 to 18 hours as determined from a single dose, fasted, human pharmacokinetic study.

9. The dose for use according to any one of claims 1 to 8, wherein once daily administration of said dose provides a steady state C-ave-day/C-ave-night ratio of 1.1 to 2.0.

10. The dose for use according to any one of claims 7 and 8, wherein the Cmax/Cmin ratio at steady state is 1.3-3.

11. The dose for use according to any one of claims 1 to 10, wherein the method increases walking speed.

12. The dose for use according to claim 11, wherein walking speed or ability is evaluated via at least one of the following or a combination thereof: Timed 25-Foot Walking test, Timed Up and Go, 2 minute walk test, six minute timed walk test, and/or, Twelve Item Multiple Sclerosis Walking Scale.

13. The dose for use according to any one of claims 1-12, wherein administration of the composition results in a 5%, 10%, 15%, 20%, 25%, 30%, 35%, 40%, 45%, 50%, 55%, or 60% reduction in multiple sclerosis disease walking impairment.

14. The dose for use according to any one of claims 1 to 13, wherein the amantadine is administered as amantadine hydrochloride.

## Patentansprüche

1. Dosis, umfassend (i) 220 mg bis 600 mg Amantadin oder pharmazeutisch verträgliche Salze davon und (ii) wenigstens einen Hilfsstoff, zur Verwendung bei einem Verfahren zum Verbessern des Gangs bei einem menschlichen Subjekt mit multipler Sklerose, wobei das Verfahren einmaltägliches orales Verabreichen der Dosis an das Subjekt umfasst.

2. Dosis zur Verwendung gemäß Anspruch 1, wobei die Dosis zusätzlich ein oder mehrere Arzneimittel ausgewählt aus der Gruppe bestehend aus 4-Aminopyridin, Baclofen, Dextromethorphan, Dimethylfumarat, Fingolimod, Methylphenidat, Teriflunomid, Tetrabenizin, Tizanidin und pharmazeutisch verträglichen Salzen davon umfasst.

3. Dosis zur Verwendung gemäß Anspruch 2, wobei das eine oder die mehreren Arzneimittel in einer Form mit verlängerter Freisetzung bereitgestellt ist/sind.

4. Dosis zur Verwendung gemäß einem der Ansprüche 1 bis 3, wobei wenigstens ein Hilfsstoff ein freisetzungsmodifizierter Hilfsstoff ist.

5. Dosis zur Verwendung gemäß einem der Ansprüche 1 bis 3, wobei wenigstens einer der Hilfsstoffe die Freisetzung des Amantadins oder pharmazeutisch verträglichen Salzes davon modifiziert, um eine Form mit verlängerter Freisetzung bereitzustellen, und wobei Verabreichung der Dosis a) eine Tmax für Amantadin von 5 bis 18 Stunden, b) eine Cmax von 1,0 bis 2,8 ng/ml pro mg Amantadin und c) eine AUC_{0-inf} von 40 bis 75 ng^{∗}h/ml pro mg Amantadin, wie gemessen in einer pharmakokinetischen Einzeldosisstudie am Menschen, bereitstellt.

6. Dosis zur Verwendung gemäß einem der Ansprüche 1 bis 5, wobei das Amantadin oder pharmazeutisch verträgliche Salz davon in einer Form mit verlängerter Freisetzung bereitgestellt ist.

7. Dosis zur Verwendung gemäß einem der Ansprüche 1 bis 6, wobei die Dosis am Morgen verabreicht wird und die Zusammensetzung eine mediane Tmax von 5 bis 9 Stunden, wie bestimmt aus einer pharmakokinetischen Nüchtern-Einzeldosisstudie am Menschen, bereitstellt.

8. Dosis zur Verwendung gemäß einem der Ansprüche 1 bis 6, wobei die Dosis 0 bis 4 Stunden vor der Bettruhe verabreicht wird und die Zusammensetzung eine mediane Tmax von 10 bis 18 Stunden, wie bestimmt aus einer pharmakokinetischen Nüchtern-Einzeldosisstudie am Menschen, bereitstellt.

9. Dosis zur Verwendung gemäß einem der Ansprüche 1 bis 8, wobei einmaltägliche Verabreichung der Dosis ein stationäres C-mittel-Tag/C-mittel-Nacht-Verhältnis von 1,1 bis 2,0 bereitstellt.

10. Dosis zur Verwendung gemäß einem der Ansprüche 7 und 8, wobei das Cmax/Cmin-Verhältnis im stationären Zustand 1,3-3 beträgt.

11. Dosis zur Verwendung gemäß einem der Ansprüche 1 bis 10, wobei das Verfahren die Gehgeschwindigkeit erhöht.

12. Dosis zur Verwendung gemäß Anspruch 11, wobei die Gehgeschwindigkeit oder -fähigkeit über wenigstens eines der Folgenden oder eine Kombination davon ausgewertet wird: 25-Feet-Gehtest mit Zeitvorgabe, Aufstehen und Gehen mit Zeitvorgabe, 2-Minuten-Gehtest, Gehtest mit Sechs-Minuten-Zeitvorgabe und/oder Zwölfpunkt-Multiple-Sklerose-Gehskala.

13. Dosis zur Verwendung gemäß einem der Ansprüche 1-12, wobei Verabreichung der Zusammensetzung zu einer Verringerung der Multiple-Sklerose-Erkrankung-Gehbeeinträchtigung von 5 %, 10 %, 15 %, 20 %, 25 %, 30 %, 35 %, 40 %, 45 %, 50 %, 55 % oder 60 % führt.

14. Dosis zur Verwendung gemäß einem der Ansprüche 1 bis 13, wobei das Amantadin als Amantadinhydrochlorid verabreicht wird.

## Revendications

1. Dose comprenant (i) 220 mg à 600 mg d'amantadine ou de sels pharmaceutiquement acceptables correspondants et (ii) au moins un excipient, pour une utilisation dans un procédé d'amélioration de la démarche chez un sujet humain ayant une sclérose en plaques, le procédé comprenant une administration par voie orale de la dose audit sujet une fois par jour.

2. Dose pour une utilisation selon la revendication 1, la dose comprenant de plus un ou plusieurs médicaments choisis dans le groupe constitué par la 4-aminopyridine, le baclofène, le dextrométhorphane, le fumarate de diméthyle, le fingolimod, le méthylphénidate, le tériflunomide, la tétrabénizine, la tizanidine, et des sels pharmaceutiquement acceptables correspondants.

3. Dose pour une utilisation selon la revendication 2, le ou les médicaments étant fournis en une forme à libération prolongée.

4. Dose pour une utilisation selon l'une quelconque des revendications 1 à 3, au moins un excipient étant un excipient de modification de libération.

5. Dose pour une utilisation selon l'une quelconque des revendications 1 à 3, au moins l'un desdits excipients modifiant la libération de l'amantadine ou d'un sel pharmaceutiquement acceptable correspondant pour fournir une forme à libération prolongée, et l'administration de la dose fournissant a) un Tmax pour l'amantadine de 5 à 18 heures, b) un Cmax de 1,0 à 2,8 ng/ml par mg d'amantadine, et c) un AUC_{0-inf} de 40 à 75 ng^{∗}h/ml par mg d'amantadine tel que mesuré dans une étude pharmacocinétique humaine à dose unique.

6. Dose pour une utilisation selon l'une quelconque des revendications 1 à 5, ladite amantadine ou un sel pharmaceutiquement acceptable correspondant étant fournie en une forme à libération prolongée.

7. Dose pour une utilisation selon l'une quelconque des revendications 1 à 6, ladite dose étant administrée le matin et la composition fournissant un Tmax médian de 5 à 9 heures tel que déterminé à partir d'une étude pharmacocinétique humaine, à jeun, à dose unique.

8. Dose pour une utilisation selon l'une quelconque des revendications 1 à 6, ladite dose étant administrée 0 à 4 heures avant le coucher et la composition fournissant un Tmax médian de 10 à 18 heures tel que déterminé à partir d'une étude pharmacocinétique humaine, à jeun, à dose unique.

9. Dose pour une utilisation selon l'une quelconque des revendications 1 à 8, une administration une fois par jour de ladite dose fournissant un rapport C-moy-jour/C-moy-nuit à l'état stable de 1,1 à 2,0.

10. Dose pour une utilisation selon l'une quelconque des revendications 7 et 8, le rapport Cmax/Cmin à l'état stable étant de 1,3 à 3.

11. Dose pour une utilisation selon l'une quelconque des revendications 1 à 10, le procédé augmentant la vitesse de marche.

12. Dose pour une utilisation selon la revendication 11, la vitesse ou la capacité de marche étant évaluée via au moins l'un des suivants ou une combinaison correspondante : essai chronométré de marche de 25 pieds, lever et marche chronométrés, essai de 2 minutes de marche, essai chronométré de six minutes de marche, et/ou échelle de marche de sclérose en plaques en douze points.

13. Dose pour une utilisation selon l'une quelconque des revendications 1 à 12, l'administration de la composition résultant en une réduction de 5 %, 10 %, 15 %, 20 %, 25 %, 30 %, 35 %, 40 %, 45 %, 50 %, 55 %, ou 60 % de l'altération de la marche due à la maladie de type sclérose en plaques.

14. Dose pour une utilisation selon l'une quelconque des revendications 1 à 13, l'amantadine étant administrée en tant que chlorhydrate d'amantadine.
